# EUROPEAN PATENT APPLICATION

(11) **EP 4 238 986 A1**
(43) Date of publication of application: **06.09.2023**
(21) Application number: 20959641.0
(22) Date of filing: 30.12.2020
(51) Int. Cl.: C07K 16/00, C40B 40/10

(54) **FC MONOMER POLYPEPTIDE AND APPLICATION THEREOF**

(30) Priority: 27.10.2020 CN 202011161007
(71) Applicant: Suzhou Forlong Biotechnology Co., Ltd., Jiangsu 215000 (CN)
(72) Inventor: YING, Tianlei, Shanghai 200433 (CN); WANG, Chunyu, Shanghai 200433 (CN)
(74) Representative: V.O.
(86) International application number: PCT/CN2020/141250
(87) International publication number: WO 2022/088484

(57) **Abstract**

Provided is an Fc monomer polypeptide, comprising CH2 and CH3 domains, the Fc monomer polypeptide comprising an amino acid sequence shown in SEQ ID NO: 1; X0 is L or S; X1 is any one amino acid among C, G, S, L, N, D, F, I, V, Y, Q, K, E, M, T or R; X2 is any one amino acid among H, L, Q, N, D, Y, R, C, G, S, F, T, I, V, A, K or M; X3 is any one amino acid among P, N, T, I, S, M, Q, R, L, G, V, A, E, D, Y, F, H or K; X4 is any one amino acid among K, N, S, I, M, E, Q, L, V, A, H, D, Y, F or T; and X5 is M or Y Beneficial effects: a class of IgG Fc monomer polypeptides is obtained, the molecular weight thereof is only half of that of a wild type Fc region, the FcRn binding function of an antibody is reserved, and efficient expression in prokaryotic cells can be achieved.

## Description

### Cross Reference to Related Applications

The present application claims the priority of CN Patent Application No. 2020111610073 filed with the China National Intellectual Property Administration on October 27, 2020, entitled "Fc Monomer Polypeptide and Application Thereof', the entire content of which is incorporated into the present application by reference.

### Technical Field

The present invention belongs to the field of biotechnology, and particularly relates to an Fc mutant monomer, an isolated CH₃ domain, and a fusion formed from one of the two with a fusion partner; a nucleic acid, plasmid, and host cell required for constructing the above Fc mutant monomer, CH₃ domain, and fusion; a corresponding pharmaceutical composition; and the use thereof.

### Background Art

A conventional antibody basic unit structure consists of four peptide chains, including two heavy chains and two light chains, which are bonded together by disulfide bonds. The shape of an antibody looks like the letter Y, and the hinge region of the Y structure is flexible. Each peptide chain has a constant region and a variable region, wherein the constant region is highly conserved in all antibodies, such as regions CH1, CH2, and CH3, which are related to the structure and effector functions of an antibody, and the variable region is specific in each antibody and can specifically bind to an antigen. An Fc segment of an antibody only comprises a constant region of a heavy chain, and a Fab segment comprises a constant region, a variable region of the heavy chain, and a variable region of a light chain. The Fc region of an antibody molecule can play a series of biological functions to further enhance the efficacy of a monoclonal antibody, and can bind to a neonatal Fc receptor (FcRn) in a pH-dependent manner, that is, the Fc region of the antibody binds to FcRn in the acidic environment of an endosome, and is circulated, transported to the cell surface and released to the external environment, so as to prevent the antibody from being degraded by the endosome, thus prolonging the half-life of the antibody. Therefore, the fusion of the Fc region with a fusion partner (such as a heterologous protein, an adhesion molecule, a nucleic acid molecule, a small molecule compound, a virus, a toxin, an immune cell, or a detectable label) can prolong the half-life of the corresponding molecule *in vivo.*

The constant region Fc of a human antibody is a homodimer, and its own molecular weight is 60 kDa. If a protein molecule is fused, the fusion protein is mostly also in the form of a dimer, which tends to increase the molecular weight of the fusion protein by several times and, meanwhile, the dimerized drug molecules may interfere with one another to affect the drug effect. For a long time, people have been exploring "antibody fragments" with a smaller molecular weight and soluble expression in prokaryotic cells as a new generation of antibody drugs with low production costs and strong tissue penetration capabilities (HOLLIGER P. et al., Nature Biotechnology, 2005, 23 (9): 1126-1136; and SAERENS D. et al., Current Opinion Pharmacology, 2008, 8 (5): 600-608), such as Fab, single-chain antibody (scFv), and nanobody. However, in order to reduce the molecular weight, these antibody fragments all give up the Fc region of IgG so that they cannot bind to FcRn, resulting in an extremely short half-life (within two hours) *in vivo,* which makes it difficult for the current small molecular weight antibody fragments to be truly used as drugs in clinical treatment of diseases.

Studies have reported that a mutant monomer molecule (mFc, sFc) of IgG1 Fc has been constructed, the molecular weight of which is only half of that of the wild-type IgG1 Fc region, and which retains the FcRn binding property and Protein A/G binding property of the antibody Fc region and has the excellent characteristics of high soluble expression in *Escherichia coli,* the half-life of which can reach approximately up to two days in animals (YING, Tianlei, et al., MAbs, 2014, 6 (5): 1201-1210; YING, Tianlei et al., JOURNAL OF BIOLOGICAL CHEMISTRY, 2012, 287 (23): 19399-19408; WO 2013/138643 A1; WANG, Chunyu et al., Front Immunol. 2017 Nov 13; 8:1545). However, only a few IgG1 Fc monomer mutants were obtained by means of previous screening methods.

In the present invention, by in-depth analysis of antibody constant regions on the basis of the previous studies, we have found that the dimerization of IgG1, IgG2, IgG3 and IgG4 Fc is mainly caused by a hydrophobic interface in the CH3 domain, including the interaction formed by at least 16 residues in each polypeptide chain. Destruction of such a strong interaction leads to the exposure of the hydrophobic surface, causing the deterioration of the solubility, stability and/or aggregation of the Fc monomer. Using molecular dynamics simulation, we innovatively constructed a large phage display library by using new synthetic biology technology, and developed a new method of antigenic solid-phase adsorption screening, while increasing the library diversity. By means of the above new technology, we obtained a class of IgG Fc monomers, which not only greatly increased the number of Fc monomers, but also all maintained the excellent properties of the previous monomeric Fc, providing sufficient choices for the development of novel antibodies. In addition, it is also confirmed that a fusion protein of a small molecular antibody fragment and an antibody IgG Fc monomer has better feasibility.

Therefore, such an IgG Fc monomer is of great significance, which can be used as a small molecular weight long-acting monomer module to fuse with an antibody fragment to construct a novel genetically engineered antibody, providing important theoretical basis and solution for breaking through the bottleneck of antibody drug development; or the IgG Fc monomer is fused or coupled with various proteins, polypeptides, small molecules, nucleic acids, etc., which target various targets, so that the fused or coupled molecules have the properties of pH-dependent binding to FcRn, thus having a potentially better developability and a longer half-life *in vivo.*

### Summary of the Invention

A main object of the present application is to optimize the mutation site by means of new synthetic biology technology, innovatively develop a new method of antigenic solid-phase adsorption screening, and obtain a novel class of Fc monomers which not only maintains the excellent properties of the antibody Fc, but also has a better feasibility in terms of fusion with a small molecular antibody fragment. This novel Fc monomer can be used as a small molecular weight long-acting monomer module that fuses with an antibody fragment to construct a novel genetically engineered antibody, thereby providing an important theoretical basis and solution for breaking through the bottleneck of antibody drug development; in addition, this novel Fc monomer may also be fused or coupled with various heterologous proteins, adhesion molecules, nucleic acid molecules, small molecule compounds, viruses, toxins, immune cells, detectable labels, etc., so that the fused or coupled molecules have the properties of pH-dependent binding to FcRn, thus having a potentially better developability and a longer half-life *in vivo.*

In order to achieve the above object, the present invention provides the following technical solution:
In a first aspect of the present invention, provided is an Fc monomer polypeptide comprising CH2 and CH3 domains, wherein the Fc monomer polypeptide comprises an amino acid sequence set forth in SEQ ID NO: 1, wherein X0 is L or S; X1 is any of amino acid C, G, S, L, N, D, F, I, V, Y, Q, K, E, M, T, or R; X2 is any of amino acid H, L, Q, N, D, Y, R, C, G, S, F, T, I, V, A, K, or M; X3 is any of amino acid P, N, T, I, S, M, Q, R, L, G, V, A, E, D, Y, F, H, or K; X4 is any of amino acid K, N, S, I, M, E, Q, L, V, A, H, D, Y, F, or T; and X5 is M or Y

As a preferred embodiment of the above Fc monomer polypeptide, the sequence of the Fc monomer polypeptide further comprises an amino acid sequence set forth in SEQ ID NO: 2; preferably, the sequence of the Fc monomer polypeptide further comprises an amino acid sequence set forth in SEQ ID NO: 3; more preferably, the sequence of the Fc monomer polypeptide further comprises an amino acid sequence set forth in SEQ ID NO: 4; further preferably, the sequence of the Fc monomer polypeptide further comprises an amino acid sequence set forth in SEQ ID NO: 5.

In a second aspect of the present invention, provided is an isolated CH3 domain, comprising amino acids at positions 113-217 in the amino acid sequence of SEQ ID NO: 1 as defined in claim 1; preferably, further comprising amino acids at positions 113-217 in the amino acid sequence of SEQ ID NO: 2 as defined in claim 2; more preferably, further comprising amino acids at positions 113-217 in the amino acid sequence of SEQ ID NO: 3 as defined in claim 2; further preferably, further comprising amino acids at positions 113-216 in the amino acid sequence of SEQ ID NO: 4 as defined in claim 2; and more further preferably, further comprising amino acids at positions 113-217 in the amino acid sequence of SEQ ID NO: 5 as defined in claim 2.

In a third aspect of the present invention, provided is a fusion formed from a fusion partner connected to the N-terminus and/or C-terminus of the peptide chain of the Fc monomer polypeptide according to one of claims 1 and 2 or the CH3 domain according to claim 3; preferably, the fusion partner is at least one of a heterologous protein, an adhesion molecule, a nucleic acid molecule, a small molecule compound, a toxin, an immune cell, and a detectable label; more preferably, the heterologous protein is at least one of an antibody, an antigen, a cytokine, a soluble receptor or a target binding region thereof fusion, a biological enzyme, a peptide, and a protein domain; further preferably, the antigen is derived from an adeno-associated virus; more further preferably, the immune cell is a chimeric antigen receptor T cell; and preferably, the fusion further comprises an amino acid sequence set forth in SEQ ID NO: 6, an amino acid sequence set forth in SEQ ID NO: 7, or an amino acid sequence set forth in SEQ ID NO: 8.

As a preferred embodiment, the fusion is formed from an Fc monomer polypeptide fused with a heterologous protein, wherein the heterologous protein is at least one of an antibody, an antigen, a cytokine, a soluble receptor domain, a ligand, an enzyme, a peptide, and a protein domain;
as a preferred embodiment, the fusion is formed from an Fc monomer polypeptide or CH3 domain fused with an antibody, wherein the antibody includes, but is not limited to, a single chain antibody (scFv), a nanobody (Nb), a bispecific antibody, a trispecific antibody, a tetraspecific antibody, bis-scFv, an antibody heavy chain variable region (VH), an antigen-binding fragment (Fab, Fab', or (Fab')2), or an Fv region (variable fragment), and the antigen is derived from an adeno-associated virus; and
as a preferred embodiment, the fusion is formed from an Fc monomer polypeptide or CH3 domain fused with an antigen, wherein the source of the antigen includes, but is not limited to, cancer, infectious diseases (such as viral, bacterial, fungal, and parasitic infections), autoimmune diseases, and inflammatory disorders.

The fusion constructed by the fusion of the Fc monomer polypeptide or CH3 domain with the antibody can target an antigen, including, but not limited to, the following listed proteins, and subunits, domains, motifs and epitopes of the following listed proteins: CD2, CD3, CD3E, CD4, CD11, CD11a, CD14, CD16, CD18, CD19, CD20, CD22, CD23, CD25, CD28, CD29, CD30, CD32, CD33 (p67 protein), CD38, CD40, CD40L, CD52, CD54, CD56, CD80, CD147, GD3, IL-1, IL-1R, IL-2, IL-2R, IL-4, IL-5, IL-6, IL-6R, IL-8, IL-12, IL-15, IL-18, IL-23, α-interferon, β-interferon, γ-interferon, TNF-α, TNFβ2, TNFc, TNFαβ, TNF-RI, TNF-RII, FasL, CD27L, CD30L, 4-1BBL, TRAIL, RANKL, TWEAK, APRIL, BAFF, LIGHT, VEGI, OX40L, TRAIL receptor-1, A1 adenosine receptor, lymphotoxin β receptor, TACI, BAFF-R, EPO, LFA-3, ICAM-1, ICAM-3, EpCAM, β1-integrin, β2-integrin, α4/β7-integrin, α2-integrin, α3-integrin, α4-integrin, α5-integrin, α6-integrin, αv-integrin, αVβ3-integrin, FGFR-3, keratinocyte growth factor, VLA-1, VLA-4, L-selectin, anti-idiotype antibody (anti-id), E-selectin, HLA, HLA-DR, CTLA-4, T cell receptor, B7-1, B7-2, VNR integrin, TGF-β1, TGF-β2, eotaxin 1, BLyS (B-lymphocyte stimulator), complement C5, IgE, factor VII, CD64, CBL, NCA 90, EGFR (ErbB-1), Her2/neu (ErbB-2), Her3 (ErbB-3), Her4 (ErbB-4), tissue factor, VEGF, VEGFR, endothelin receptor, VLA-4, hapten NP-capped or NIP-capped protein, T cell receptor α/β, E-selectin, digoxin, placental alkaline phosphatase (PLAP) and testicular PLAP-like alkaline phosphatase, transferrin receptor, carcinoembryonic antigen (CEA), CEACAM5, HMFG PEM, mucin MUC1, MUC18, heparanase I, human cardiac myosin, tumor-associated glycoprotein-72 (TAG-72), tumor-associated antigen CA125, prostate-specific membrane antigen (PSMA), high molecular weight melanoma-associated antigen (HMW-MAA), cancer-associated antigen, Gcoprotein IIb/IIIa (GPIIb/IIIa), tumor-associated antigen expressing Lewis Y related carbohydrate, human cytomegalovirus (HCMV) gH envelope glycoprotein, HIV gp120, HIV gp140, HCMV, respiratory syncytial virus RSVF, RSVF Fgp, VNR integrin, IL-8, cytokeratin tumor-associated antigen, Hep B gp120, CMV, gpIIbIIIa, HIV IIIB gp120V3 loop, respiratory syncytial virus (RSV) Fgp, herpes simplex virus (HSV) gD glycoprotein, HSV gB glycoprotein, HCMV gB envelope glycoprotein and *Clostridium perfrmgens* toxin, and programmed death-1 (PD-1).

The targets listed above refer not only to specific proteins and biomolecules, but also to biochemical pathways or various pathways containing same. For example, reference to CTLA-4 as a target antigen means that ligands and receptors constitute T cell costimulatory pathways, including CTLA-4, B7-1, B7-2, and CD28, and any other undiscovered ligands or receptors are also targets. Therefore, the targets used herein refer not only to a specific biomolecule, but also to a group of proteins that interact with the target and the members of the biochemical pathway to which the target belongs. It should be further understood by those skilled in the art that any of the above-mentioned target antigens, ligands or receptors connected thereto, or other members of their corresponding biochemical pathways can be operably linked to the Fc monomer polypeptide of the present invention to produce Fc fusions. Therefore, by way of example, an EGFR-targeting Fc fusion can be constructed by operably linking an Fc monomer polypeptide with EGF, TGF-α, or any other ligand that can bind to EGFR, whether discovered or not. Therefore, the Fc variant of the present invention can be operably linked to EGFR to produce an Fc fusion that can bind to EGF, TGFα, or any other ligand that can bind to EGFR, whether discovered or not. Therefore, in fact, any polypeptides, whether they are ligands, receptors, or some other proteins or protein domains, including, but not limited to, the above targets and proteins constituting their corresponding biochemical pathways, can be operably linked to the Fc monomer polypeptide of the present invention to develop Fc fusions.

The antigen may be derived from a virus. The virus described in the present invention is a virus, for example, from one of the following families: the Retroviridae family (e.g., human immunodeficiency virus (HIV) and human T cell leukemia virus (HTLV)); the Picornaviridae family (e.g., poliovirus, hepatitis A virus, hepatitis C virus, enterovirus, human coxsackie virus, rhino virus, echo virus, and foot-and-mouth disease virus); the Calmyxoviridae family (such as virus strains that cause gastroenteritis); the Togaviridae family (e.g., equine encephalitis virus and rubella virus); the Flaviviridae family (e.g., dengue virus, yellow fever virus, West Nile virus, St. Louis encephalitis virus, Japanese encephalitis virus, and other encephalitis viruses); the Coronaviridae family (e.g., coronavirus and severe acute respiratory syndrome (SARS) virus); the Rhabdoviridae family (e.g., vesicular stomatitis virus and rabies virus); the Paramyxoviridae family (e.g., parainfluenza virus, mumps virus, measles virus, and respiratory syncytial virus (RSV)); the Orthomyxoviridae family (e.g., influenza virus); the Bunyaviridae family (e.g., Hantavirus, Sin Nombre virus, Rift Valley fever virus, bunya virus, phleboviruses, and Nairo virus); the Arenaviridae family (e.g., hemorrhagic fever virus, Machupo virus, and Junin virus); the Reoviridae family (e.g., reovirus, orbiviurse, and rotavirus); the Bimaviridae family; the Hepadnaviridae family (e.g., hepatitis B virus); the Parvoviridae family (e.g., parvovirus); the Papovaviridae family (e.g., papillomavirus, polyoma virus, and BK virus); the Adenoviridae family (e.g., most adenoviruses, such as adeno-associated viruses); the Herpesviridae family (e.g., herpes simplex virus (HSV-1 and HSV-2), cytomegalovirus (CMV), Epstein-Barr virus (EBV), varicella zoster virus (VZV), and other herpes viruses, including HSV-6); the Poxviridae family (e.g., smallpox virus, vaccinia virus, and poxvirus); the Iridoviridae family (such as African swine fever virus); the Viraceae family (e.g., Ebola virus and Marburg virus); the Caliciviridae family (e.g., Norwalk virus); and unclassified viruses (e.g., the pathogen of spongiform encephalopathy, the pathogen of hepatitis delta (considered as a defective satellite of hepatitis B virus), and astroviruses).

The antigen may be from a bacterium. The bacteria described in the present invention are, for example, *Helicobacter pylori*, *Borelia burgdorferi*, *Legionella pneumophila*, mycobacteria (such as *M. tuberculosis*, *M. avium*, *M. intracellulare*, *M. kansaii*, and *M. gordonae), Staphylococcus aureus, Neisseria gonorrhoeae, Neisseria meningitidis, Listeria monocytogenes*, *Streptococcus pyogenes* (group A streptococcus), *Streptococcus agalactiae* (group B streptococcus), *Streptococcus* (the Viridans group streptococci), *Streptococcus faecalis*, *Streptococcus bovis*, *Streptococcus* (anaerobic bacteria), *Streptococcus pneumoniae*, pathogenic *Campylobacter*, *Enterococcus*, *Haemophilus influenzae*, *Bacillus anthracis*, *Corynebacterium diphtheriae*, *Corynebacterium*, classical swine fever virus, *Clostridium perfringens*, *Clostridium tetani*, *Enterobacter aerogenes*, *Klebsiella pneumoniae*, *Pasteurella multocida, Bacteroides, Clostridium, Streptomyces maltophilia, Treponema pallidum*, *Treponema*, *Leptospira*, or *Actinomyces israelli.*

The antigen may be from a fungus. The fungi described in the present invention are, for example, *Cryptococcus neoformans*, *Histoplasma capsulatum, Coccidioides immitis, Blastomyces dermatitidis*, *Chlamydia trachomatis*, or *Candida albicans.*

The antigen may be from a parasite. The parasite described in the present invention is, for example, *Plasmodium falciparum* or *Toxoplasma gondii.*

The antigen may be a cancer antigen, such as a solid tumor or a hematogenous carcinoma antigen. The solid tumor described in the present invention is sarcoma or cancer, such as fibrosarcoma, myxosarcoma, liposarcoma, chondrosarcoma, osteogenic sarcoma, or another sarcoma, synovioma, mesothelioma, Ewing's tumor, leiomyosarcoma, rhabdomyosarcoma, colon cancer, lymphatic malignancy, pancreatic cancer, breast cancer, lung cancer, ovarian cancer, prostate cancer, hepatocellular carcinoma, squamous cell carcinoma, basal cell carcinoma, adenocarcinoma, hidradenocarcinoma, sebaceous gland carcinoma, papillary carcinoma, papillary adenocarcinoma, medullary carcinoma, bronchial carcinoma, renal cell carcinoma, hepatocellular carcinoma, cholangiocarcinoma, choriocarcinoma, nephroblastoma, cervical carcinoma, testicular tumor, bladder cancer, or central nervous system tumor (such as glioma, astrocytoma, medulloblastoma, craniopharyngioma, ependymoma, pineal gland, hemangioblastoma, acoustic neuroma, oligodendroglioma, hemangioma, melanoma, neuroblastoma, or retinoblastoma). The hematogenous carcinoma described in the present invention is leukemia, such as acute leukemia (such as acute lymphocytic leukemia, acute myelogenous leukemia, acute myeloid leukemia, and myeloblastic, promyelocytic, myelomonocytic, monocytic and erythroleukemia); and chronic leukemia (such as chronic myelocytic (granulocytic) leukemia, chronic granulocytic leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia, or myelodysplasia. Tumor antigens are well known in the art, including, for example, carcinoembryonic antigen (CEA), β-human chorionic gonadotropin (β-HCG), alpha-fetoprotein (AFP), lectin-reactive AFP, (AFP-L3), thyroglobulin, RAGE-1, MN-CA IX, human telomerase reverse transcriptase (hTERT), RU1, RU2 (AS), intestinal carboxyl esterase, mut hsp70-2, M-CSF, prostaglandin enzyme, prostate-specific antigen (PSA), PAP, NY-ESO-1, LAGE-1a, p53, prostein, PSMA, Her2/neu, survivin and telomerase, prostate cancer tumor antigen-1 (PCTA-1), melanoma-associated antigen (MAGE), ELF2M, neutrophil elastase, ephrin B2, and CD22. It can also be any cancer-related protein, such as IGF-I, IGF-II, IGR-IR, or mesothelin.

As a preferred embodiment, the fusion is formed from an Fc monomer polypeptide or CH3 domain fused with a cytokine.

The cytokines described in the present invention include, but are not limited to, growth hormones (human growth hormone, N-methionyl human growth hormone, and bovine growth hormone), glycoprotein hormones (parathyroid hormone, thyroxine, insulin, proinsulin, relaxin, prorelaxin, follicle-stimulating hormone (FSH), thyrotropin (TSH), and luteinizing hormone (LH)), liver growth factor, fibroblast growth factor , prolactin, galactagogin, tumor necrosis factor-α and -β (TNF-α and TNF-β), Müllerian-inhibiting substance, statin, activin, vascular endothelial growth factor, integrin, thrombopoietin (TPO), nerve growth factor NGF-β, platelet-derived growth factor, transforming growth factors TGF-α and TGF-β, insulin-like growth factor-I and -II, erythropoietin (EPO), osteoinductive factor, interferon IFN-α/β/γ, colony stimulating factors (CSFs) (macrophage-CSF (M-CSF)/granulocyte macrophage-CSF (CM-CSF)/granulocyte-CSF (G-CSF)), interleukins (ILs) IL-1/1α/2/3/4/5/6/7/8/9/10/11/12/15, and other polypeptide factors.

As a preferred embodiment, the fusion is formed from an Fc monomer polypeptide or CH3 domain fused with a soluble receptor or a target binding region thereof. The soluble receptor includes, but is not limited to, tumor necrosis factor receptor, thrombopoietin receptor c-Mpl, IL-1R1 and IL-1 receptor accessory protein (IL-1RAcP), IL-4 receptor, vascular endothelial growth factor receptor (VEGFR), epidermal growth factor receptor (EGFR), human epidermal growth factor receptor 2 (abbreviated as HER2), platelet-derived growth factor receptor (PDGFR), and fibroblast growth factor receptor (FGFR).

As a preferred embodiment, the fusion is formed from an Fc monomer polypeptide or CH3 domain in association with an antibody-directed enzyme prodrug therapy (ADEPT). ADEPT can be carried out by coupling or operably linking the Fc monomer polypeptide or CH3 domain to a prodrug activating enzyme that can convert the prodrug into an activated anticancer drug. The enzyme component of an immunoconjugate for ADEPT includes any enzyme that can activate the prodrug in such a way that the prodrug can be converted into a more active and cytotoxic form. The enzymes used in the method of the present invention include, but are not limited to, alkaline phosphatase for transforming a phosphate-containing prodrug into a free drug; arylsulfatase for transforming a sulfate-containing prodrug into a free drug; cytidine deaminase for transforming nontoxic 5-fluorocytosine into an anticancer drug (5-fluorouracil); proteases that can be used for transforming a peptide-containing prodrug into a free drug, e.g., serratiopeptidase, thermolysin, subtilisin, carboxypeptidase, and cathepsin (such as cathepsins B and L); D-alanyl carboxypeptidase for transforming a prodrug containing D-amino acid substitute; sugar lyases for transforming glycosylated prodrugs into free drugs, e.g., β-galactosidase and neuraminidase; β-lactamase for transforming a α-lactam-derived drug into a free drug; and penicillin amidase for transforming a drug derived by substituting the nitrogen atom of its amine respectively with phenoxyacetyl or phenylacetyl into a free drug, such as penicillin V amidase or penicillin G amidase. Alternatively, antibodies with enzymatic activity, also known in the art as "abzymes", can be used for transforming prodrugs into active free drugs (see, for example, Massey, 1987, Nature 328: 457-458) and for preparing fusion-abzyme conjugates for delivering the abzymes to anticancer drugs in tumor cell populations.

More preferably, the fusion comprises an Fc monomer polypeptide with an amino acid sequence set forth in SEQ ID NO: 1, an amino acid sequence set forth in SEQ ID NO: 6, an amino acid sequence set forth in SEQ ID NO: 7, and an amino acid sequence set forth in SEQ ID NO: 8.

Preferably, the sequence of SEQ ID NO: 1 is as follows:
**APELLGGPSVFLFPPKPKDTLMISRTPEVTCVVVDVSHEDPEVKFNWYVDGVEVHNAKTKPREEQYNS** TYRVVSVLTVLHQDWLNGKEYKCKVSNKALPAPIEKTISKAKGQPREPQVYTXOPPSRDELTKNQVSL XlCX2VKGFYPSDIAVEWESNGOPENNYKTTX3PVLDSDGSFFLYSX4LTVDKSRWOOGNVFSCSVX5 HEALHNHYTQKSLSLSPGK;
the sequence of SEQ ID NO: 6 is as follows:

   QVQLVQSGGGLVQPGGSLRLSCAASAFDFSDYEMSWVREAPGKGLEWIGEINDSGNTIYNPSLKSRVT ISRDNSKNTLYLQMNTLRAEDTAIYYCAIYGGNSGGEYWGOGTLVTVSS;
the sequence of SEQ ID NO: 7 is as follows: 
EVQLVESGGGLVQPGGSLRLSCAASGFTFSNYGMSWVRQAPGKGLEWIGSIYYSGSTNYNPSLKSLVT
ISRDNSKNTLYLQMNSLRAEDTATYYCARESIDYWGQGTLVTVSS;
the sequence of SEQ ID NO: 8 is as follows: 
KKVVYGKKGDTVELTCTASQKKNIQFHWKNSNQIKILGNQGSFLTKGPSKLNDRVDSRRSLWDQGNFP
LIIKNLKPEDSDTYICEVEDQKEEVQLVVVG,
wherein the amino acid sequence set forth in SEQ ID NO: 6 is an anti-HIV-1 gp120 nanobody, the amino acid sequence set forth in SEQ ID NO: 7 is an anti-CD16A nanobody, and the amino acid sequence set forth in SEQ ID NO: 8 is a human immunodeficiency virus (HIV-1) gp140 protein binding fragment. The fusion is determined by the presence of an anti-CD16A heavy chain variable region and a human CD4 soluble single domain fragment and the specific gene sequence of the binding site of neonatal Fc receptor (FcRn). By using this fusion or the coding sequence therefor, various forms of genetically engineered antibodies can be transformed and produced in prokaryotic or eukaryotic cells or any expression systems, and HIV-1 virus-related diseases can be prevented or treated clinically.

As a preferred embodiment, the fusion is formed from an Fc monomer polypeptide or CH3 domain fused with a small molecule compound. The small molecule compound described in the present invention can include any therapeutic agents or analogues thereof that can direct an Fc fusion to a therapeutic target.

The target can be any molecule, preferably an extracellular receptor, which is related to diseases, and can include but not limited to one or more of GABA-targeting receptor, purinergic receptor, adrenaline receptor, histamine receptor, opioid receptor, chemical factor receptor, glutamate salt (ester) receptor, nicotine receptor, 5HT (5-hydroxytryptamine) receptor, and estrogen receptor.

As a preferred embodiment, the fusion is formed from an Fc monomer polypeptide or CH3 domain fused with an immune cell. The immune cells described in the present invention include, but are not limited to, dendritic cells (DCs), lymphokine-activated killer cells (LAKs), cytokine-induced killer cells (CIKs), DC-CIKs, natural killer cells (NKs), tumor infiltrating lymphocytes (TILs), chimeric antigen receptor engineered T cells (CAR-T), and genetically modified T cell receptor (gene modified TCR). More preferably, the fusion is formed from an Fc monomer polypeptide or CH3 domain fused with a chimeric antigen receptor T cell.

As a preferred embodiment, the fusion is formed from an Fc monomer polypeptide or CH3 domain fused with a toxin.

The toxins described in the present invention include, but are not limited to, *Pseudomonas* exotoxin (PE), ricin, abrin, *diphtheria* toxin and subunits thereof, ribonucleoprotein, ribonuclease, saporin and calicheamicin, and botulinum toxins A to F. These toxins are well known in the art, many of them are readily available, and they may also derived from commercial sources (for example, Sigma Chemical Company, St. Louis, Mo.).

As a preferred embodiment, the fusion is formed from an Fc monomer polypeptide or CH3 domain fused with a detectable label. The detectable labels described in the present invention include, but are not limited to, fluorescent labels, radioactive labels, avidin, and biotin.

The linkers described in the present invention include, but are not limited to, peptide bonds at the N- and C-termini of proteins and protein domains, disulfide bond linkers, polypeptide chains containing flexible amino acid residues, linkers naturally occurring in human bodies, chemical crosslinking agents, and non-protein polymers.

Preferably, the linker described in the present invention refers to a polypeptide chain containing flexible amino acid residues, which are Gly, Ser, Ala, or Thr. The polypeptide chain should have a suitable distance, which is suitable for linking two molecules such that they have the correct configuration relative to each other so as to maintain the desired activity. A suitable length for this purpose includes at least one and no more than 30 amino acid residues. Preferably, the length of the linker is about 1-30 amino acids, and a preferred length of the linker is 1, 2, 3, 4, 5, 6, 7, 8, 9, 10, 11, 12, 13, 14, 15, 16, 17, 18, 19, and 20 amino acids. In addition, the amino acid residues chosen to be included in the linker should not exhibit properties that significantly affect the activities of the two linked molecules. Therefore, the linker generally does not show charges inconsistent with the two linked molecules, or does not affect internal folding, or forms bonding or other interactions with amino acid residues in one or more monomers, which will seriously hinder the binding of receptor monomer domains. The linker containing flexible amino acid residues includes glycine-serine polymers (such as (GS)n, (GSGGS)n (SEQ ID NO: 9), (GGGGS)n (SEQ ID NO: 10), and (GGGS)n (SEQ ID NO: 11), wherein n is an integer of at least 1), glycine-alanine polymers, alanine-serine polymers, as well as other flexible linkers known in the art, such as a linker sequence of Shaker potassium channels, etc.

Preferably, the linker described in the present invention refers to a linker naturally occurring in the human body, including, but not limited to, a sequence containing an antibody hinge region, i.e., a sequence linking Fab and Fc regions of an antibody; or a sequence substantially similar to the hinge region of the antibody.

Preferably, the linker described in the present invention refers to a chemical crosslinking agent, including but not limited to a bifunctional protein coupling agent, such as N-succinyl-3-(2-pyridyldithio)-propionate (SPDP), succinyl-4-(N-maleimidomethyl)cyclohexane-1-carboxylate, and iminothiolane (IT); bifunctional derivatives of imidates, such as dimethyl adipimidate hydrochloride; activated esters, such as disuccinyl suberate; aldehydes, such as glutaraldehyde; diazido compounds, such as bis-(p-azidobenzoyl)-hexamethylenediamine; diazo derivatives, such as bis-(p-diazobenzoyl)-ethylenediamine; diisocyanates such as toluene-2,6-diisocyanate; and bi-activated fluorine compounds, such as 1,5-difluoro-2,4-dinitrobenzene.

Preferably, the linker described in the present invention refers to a non-protein polymer, including but not limited to polyethylene glycol (PEG), polypropylene glycol, polyoxyalkylene, or a copolymer of polyethylene glycol and polypropylene glycol.

In a fourth aspect of the present invention, provided is a nucleic acid molecule, which encodes the Fc monomer polypeptide according to claim 1 or 2, the CH3 domain according to claim 3, and/or the fusion according to claim 4.

In a fifth aspect of the present invention, provided is a plasmid comprising the nucleic acid molecule according to claim 5.

In a sixth aspect of the present invention, provided is a host cell comprising the plasmid according to claim 6. The host cell provided by the present invention can be any prokaryotic cell or eukaryotic cell, including, but not limit to, bacterial cells (e.g., *Escherichia coli*), insect cells (e.g., using a baculovirus expression system), yeast or mammalian cells (e.g., CHO or BHK cell lines), and other cells well known to those skilled in the art. Due to the short culture period and low production costs, *Escherichia coli* can be used as a preferred host cell.

In a fourth aspect of the present application, provided is a nucleic acid molecule, which encodes the Fc monomer polypeptide according to claim 1 or 2, the CH3 domain according to claim 3, and/or the fusion according to claim 4.

In a fifth aspect of the present application, provided is a plasmid comprising the nucleic acid molecule according to claim 5.

In a sixth aspect of the present application, provided is a host cell comprising the plasmid according to claim 6.

In a seventh aspect of the present application, provided is a pharmaceutical composition, comprising a prophylactically or therapeutically effective dose of the Fc monomer polypeptide according to either of claims 1 and 2, the CH3 domain according to claim 3, or the fusion according to claim 4, and a physiologically or pharmaceutically acceptable carrier; preferably, further comprising a prophylactically or therapeutically effective dose of the nucleic acid molecule according to claim 5 or the plasmid according to claim 6, and a physiologically or pharmaceutically acceptable carrier;
preferably, the composition includes but is not limited to a freeze-dried dosage form, an aqueous solution dosage form, a liposome, a capsule dosage form, etc.

The concentration of the Fc monomer polypeptide or CH3 domain or the nucleic acid molecule of the Fc monomer polypeptide or CH3 domain, the plasmid, or the fusion thereof according to the present invention can vary from about 0.1% to 100% (by weight).

The present invention provides a method for diagnosing, preventing or treating a disease, comprising administering the fusion, nucleic acid molecule, plasmid and pharmaceutical composition of the present invention to a subject.

The disease described in the present invention is an autoimmune disease, an inflammatory disease, a neurodegenerative disease, a cancer, or a pathogen infection.

Preferably, the Fc monomer polypeptide of the present invention can be used for treating a cancer. The cancers described in the present invention include, but are not limited to, lymphoma, blastocytoma, sarcoma (including liposarcoma), neuroendocrine tumors, mesothelioma, schwannoma, meningioma, adenoma, melanoma, and aleukemic leukemia, or lymphatic malignancy. More specific examples of the above cancers include squamous cell carcinoma (e.g., squamous epithelial cell carcinoma), lung cancer, small cell lung cancer, non-small cell lung cancer, lung adenocarcinoma and lung squamous cell carcinoma, peritoneal cancer, hepatocellular carcinoma, gastric cancer, gastrointestinal cancer, pancreatic cancer, malignant glioma, cervical cancer, ovarian cancer, liver cancer, bladder cancer, hepatocellular carcinoma, breast cancer, colon cancer, rectal cancer, colorectal cancer, endometrial or uterine cancer, salivary gland cancer, renal cancer, prostate cancer, vulvar cancer, thyroid cancer, liver cancer, anal cancer, penile cancer, testicular cancer, esophageal cancer, bile duct tumor, head cancer, neck cancer, bone marrow stromal tumor, osteoclastoma, multiple myeloma, osteolytic bone cancers, central nervous system tumor, brain tumor (glioma, neuroblastoma, astrocytoma, medulloblastoma, ependymoma, and retinal neuroblastoma), nasopharyngeal carcinoma, basal cell carcinoma, cholangiocarcinoma, Kaposi's sarcoma, primary liver cancer or endometrial carcinoma, and vascular system tumor (angiosarcoma and hemagiopericytoma).

Preferably, the Fc monomer polypeptide of the present invention can be used for treating a pathogen infection. The pathogens described in the present invention include, but are not limited to, bacteria, fungi, viruses, and parasites.

The Fc variant of the present invention can be used for treating diseases including, but not limited to, congestive heart failure (CHF), vasculitis, rosacea, acne, eczema, myocarditis and other myocardial diseases, systemic lupus erythematosus, diabetes mellitus, spondylopathy, synovial fibroblast proliferation, bone loss, Paget's disease, disuse osteopenia, malnutrition, periodontal disease, familial spleen anemia, Langerhans cell histiocytosis, spinal cord injury, acute septic arthritis, osteomalacia, hypercortisolism, monostotic fibrous dysplasia, multiple osteofibrous dysplasia, periodontal reconstruction and fracture, sarcoidosis, bone metastasis/osteodynia treatment and humoral hypercalcemia of malignancy, ankylosing spondylitis and other spinal arthropathy, transplant rejection, virus infection, hematologic neoplasm, Hodgkin's lymphoma, and non-Hodgkin's lymphoma (Burkitt's lymphoma, small lymphocytic lymphoma/chronic lymphocytic leukemia, mycosis fungoides, mantle cell lymphoma, follicular lymphoma, diffuse giant B cell lymphoma, marginal zone lymphoma, hairy cell leukemia and lymphoplasmacytic leukemia), lymphocyte precursor cell tumor, B cell acute lymphoblastic aleukemic leukemia/lymphoma, T cell acute lymphoblastic aleukemic leukemia/lymphoma, thymoma, mature T and NK cell tumors, peripheral T cell aleukemic leukemia, mature T cell aleukemic leukemia/T cell lymphoma, large granular lymphocytic leukemia, Langerhans cell histiocytosis, myeloma of acute myelogenous granulocytic leukemia, mature acute myelogenous leukemia (AML), differentiated acute myelogenous leukemia, acute promyelocytic leukemia, acute myelomonocytic leukemia, acute monocytic leukemia, myelodysplastic syndrome, chronic myeloproliferative disease, chronic myelocytic leukemia, osteoporosis, hepatitis, HIV, AIDS, spinal arthritis, rheumatoid arthritis, inflammatory bowel disease (IBD), sepsis and septic shock, segmental ileitis, psoriasis, scleroderma, graft-versus-host disease (GVHD), allogenic islet graft rejection, hematological malignancies such as multiple myeloma (MM), myelodysplastic syndrome (MDS) and acute myelogenous leukemia (AML), and tumor-related inflammation, peripheral nerve injury, or demyelinating diseases.

In an eighth aspect of the present invention, provided is a detection kit, comprising the fusion according to claim 4, the nucleic acid molecule according to claim 5, or the plasmid according to claim 6.

In an eighth aspect of the present invention, provided is the use of the above detection kit for detecting a pathogen or a tumor cell, preferably for detecting human immunodeficiency virus HIV. The pathogens include viruses, bacteria, fungi, parasitic infections, etc. The tumor cells include various benign tumor cells, malignant tumor cells (i.e., cancer cells), solid tumor cells, and hematogenous carcinoma cells.

Compared with the prior art, the beneficial effects of the present invention lie in that an Fc monomer polypeptide is described in the present invention, and by modifying an IgG Fc region by means of antibody engineering and synthetic biology, a class of IgG Fc monomer peptide polypeptides are obtained which have a molecular weight only half of that of the wild-type Fc region, retain the FcRn binding function of an antibody, and can achieve efficient expression in a prokaryotic cell. Such an Fc monomer polypeptide can be used to fuse with various antibodies, proteins, polypeptides, small molecules, nucleic acids, etc., which target various targets, and the fusion is still a monomer molecule, which has pH-dependent FcRn binding characteristics, thus having potentially better developability and longer *in vivo* half-life.

### Brief Description of the Drawings

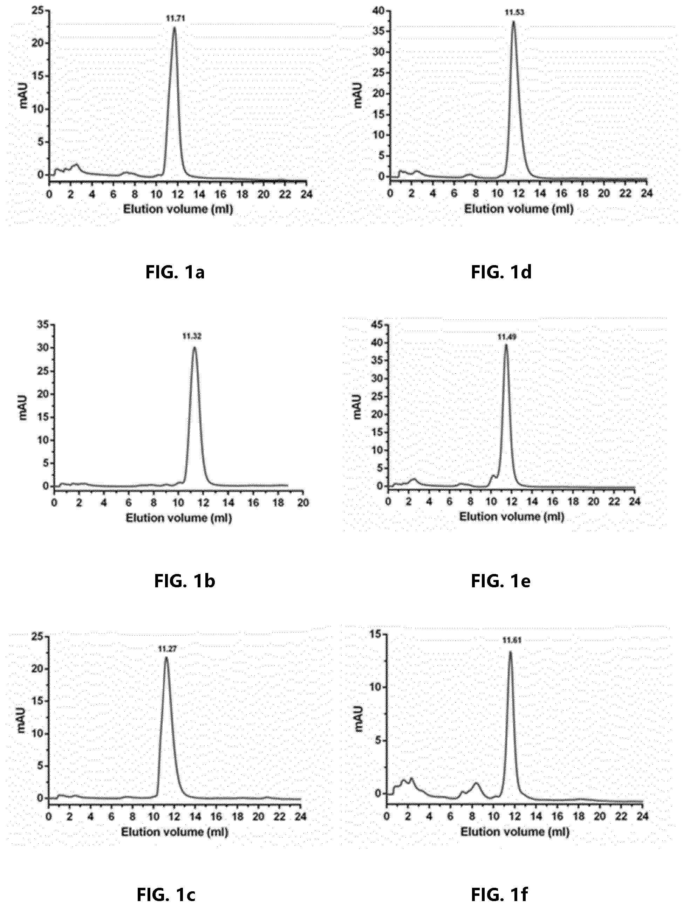

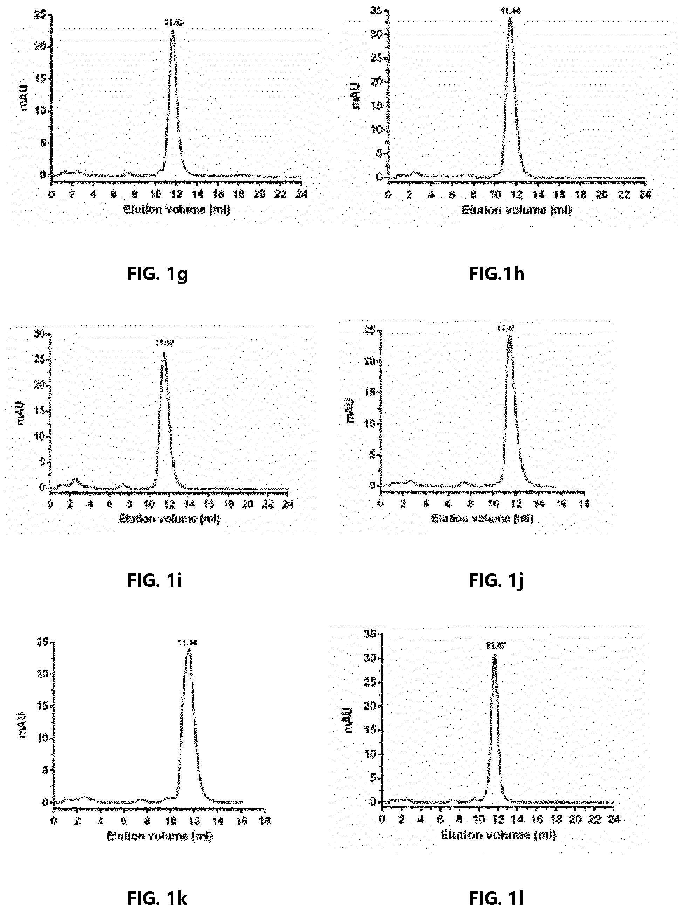

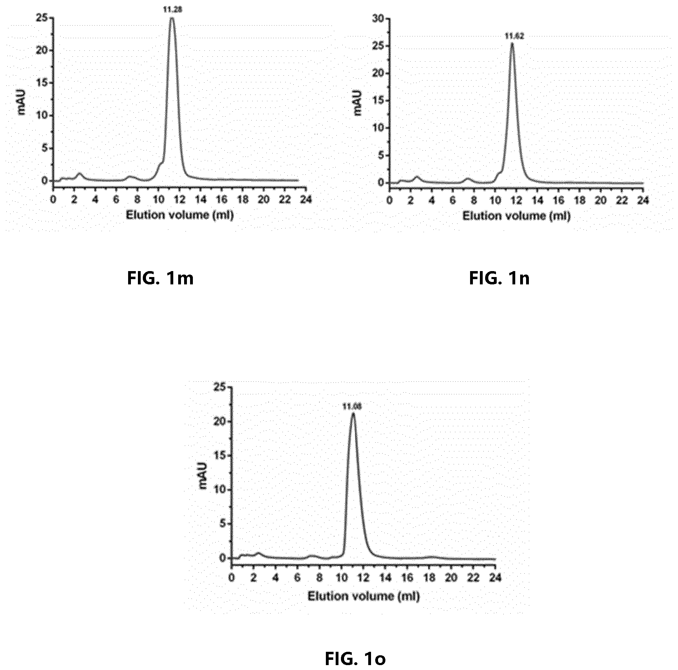

Figs. 1a-1l: the amino acid sequence set forth in SEQ ID NO: 1, wherein X0 is L; X1 is the amino acid C, G, S, L, N, D, F, I, V, Y, Q, K, E, M, or T, respectively; X2 is H; X3 is K; X4 is T; and X5 is M. By SEC, IgG1 Fc mutants are detected to be present as monomers, respectively (Fig. 1a corresponds to the case where X1 is the amino acid C, Fig. 1b corresponds to the case where X1 is the amino acid G, Fig. 1c corresponds to the case where X1 is the amino acid S, Fig. 1d corresponds to the case where X1 is the amino acid L, Fig. 1e corresponds to the case where X1 is the amino acid N, Fig. 1f corresponds to the case where X1 is the amino acid D, Fig. 1g corresponds to the case where X1 is the amino acid F, Fig. 1h corresponds to the case where X1 is the amino acid I, Fig. 1i corresponds to the case where X1 is the amino acid V, Fig. 1j corresponds to the case where X1 is the amino acid Y, Fig. 1k corresponds to the case where X1 is the amino acid Q, Fig. 1l corresponds to the case where X1 is the amino acid K, Fig. 1m corresponds to the case where X1 is the amino acid E, Fig. 1n corresponds to the case where X1 is the amino acid M, and Fig. 1o corresponds to the case where X1 is the amino acid T);

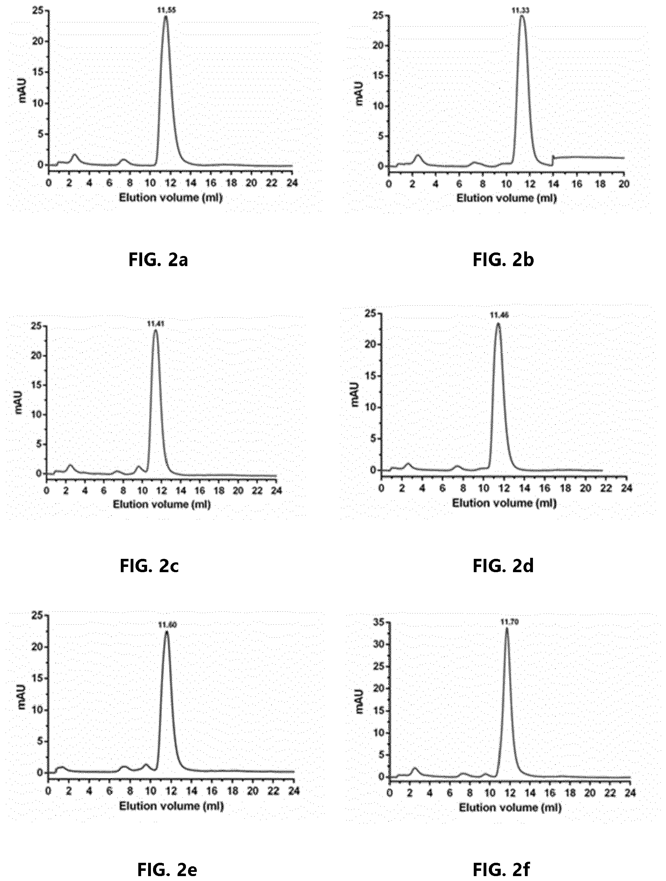

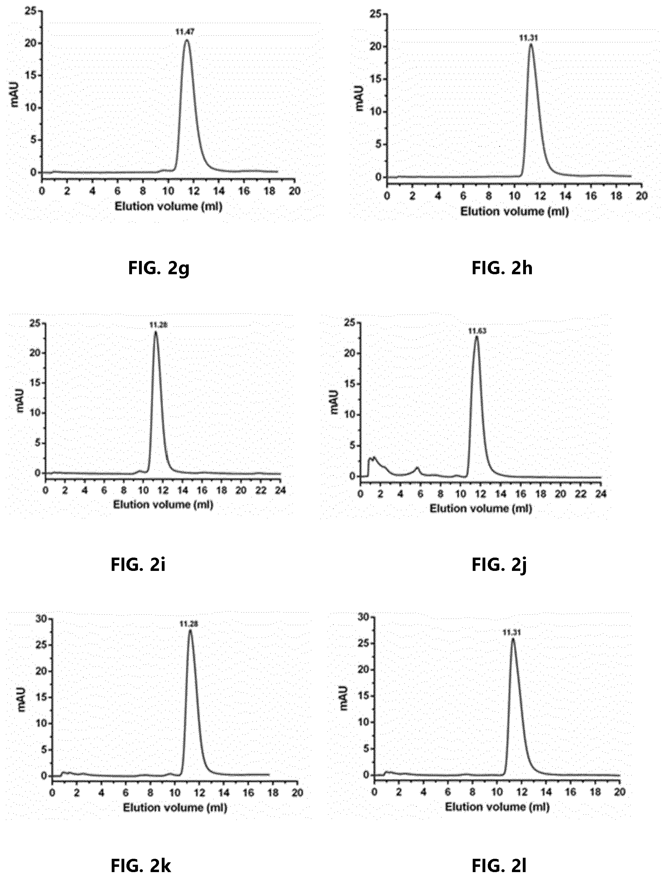

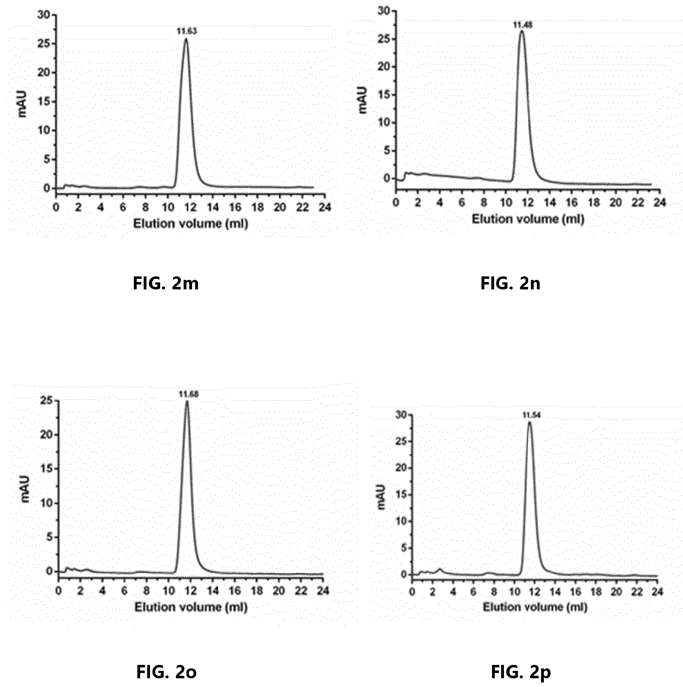

Figs. 2a-2p: the amino acid sequence set forth in SEQ ID NO: 1, wherein X0 is L; X1 is R; X2 is the amino acid L, Q, N, D, Y, R, C, G, S, F, T, I, V, A, K, or M, respectively; X3 is K; X4 is T; and X5 is M. By SEC, IgG1 Fc mutants are detected to be present as monomers, respectively (Fig. 2a corresponds to the case where X2 is the amino acid L, Fig. 2b corresponds to the case where X2 is the amino acid Q, Fig. 2c corresponds to the case where X2 is the amino acid N, Fig. 2d corresponds to the case where X2 is the amino acid D, Fig. 2e corresponds to the case where X2 is the amino acid Y, Fig. 2f corresponds to the case where X2 is the amino acid R, Fig. 2g corresponds to the case where X2 is the amino acid C, Fig. 2h corresponds to the case where X2 is the amino acid G, Fig. 2i corresponds to the case where X2 is the amino acid S, Fig. 2j corresponds to the case where X2 is the amino acid F, Fig. 2k corresponds to the case where X2 is the amino acid T, Fig. 2l corresponds to the case where X2 is the amino acid I, Fig. 2m corresponds to the case where X2 is the amino acid V, Fig. 2n corresponds to the case where X2 is the amino acid A, Fig. 2o corresponds to the case where X2 is the amino acid K, and Fig. 2p corresponds to the case where X2 is the amino acid M);

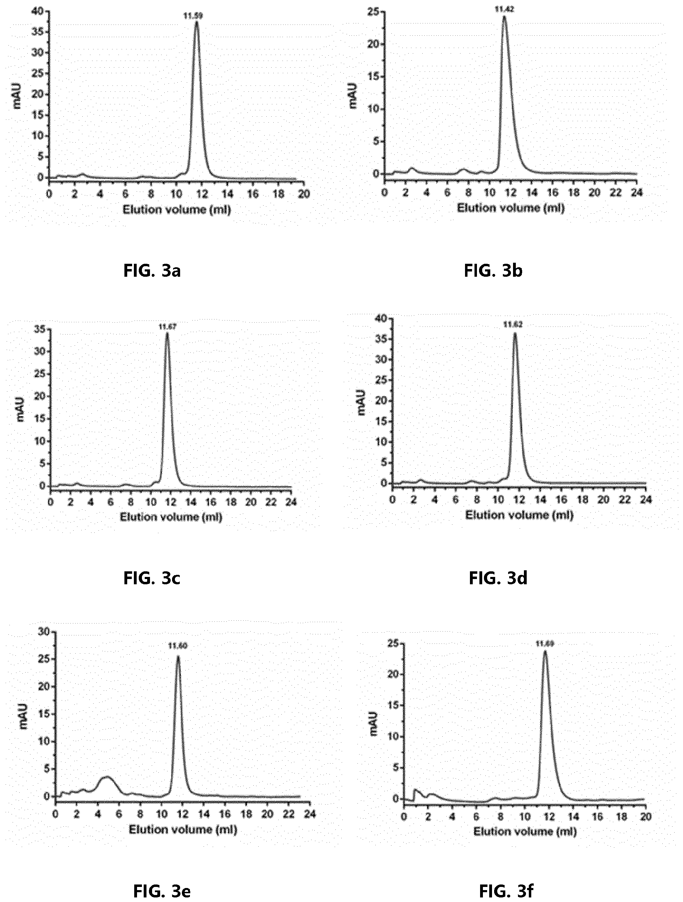

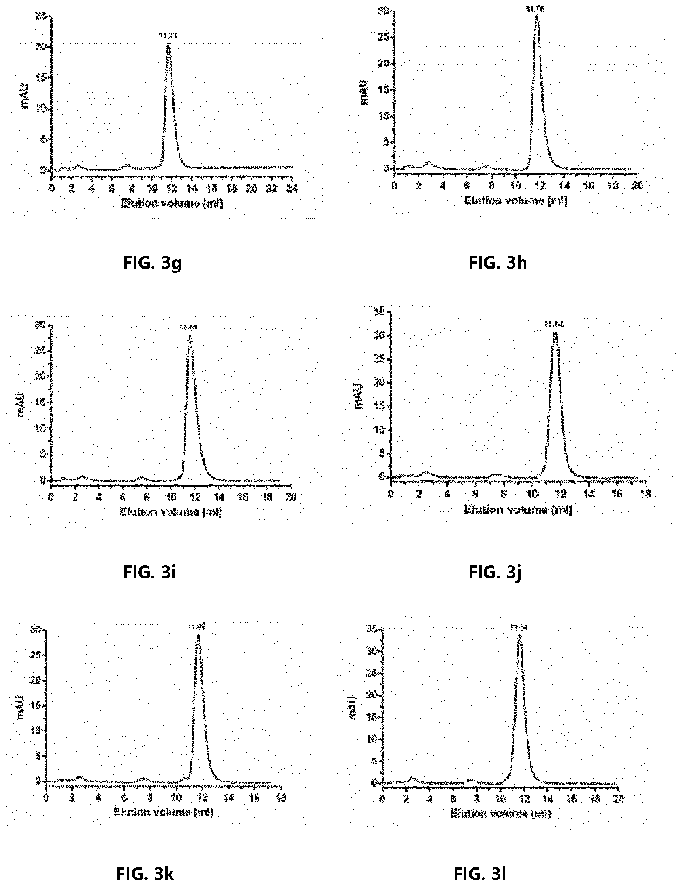

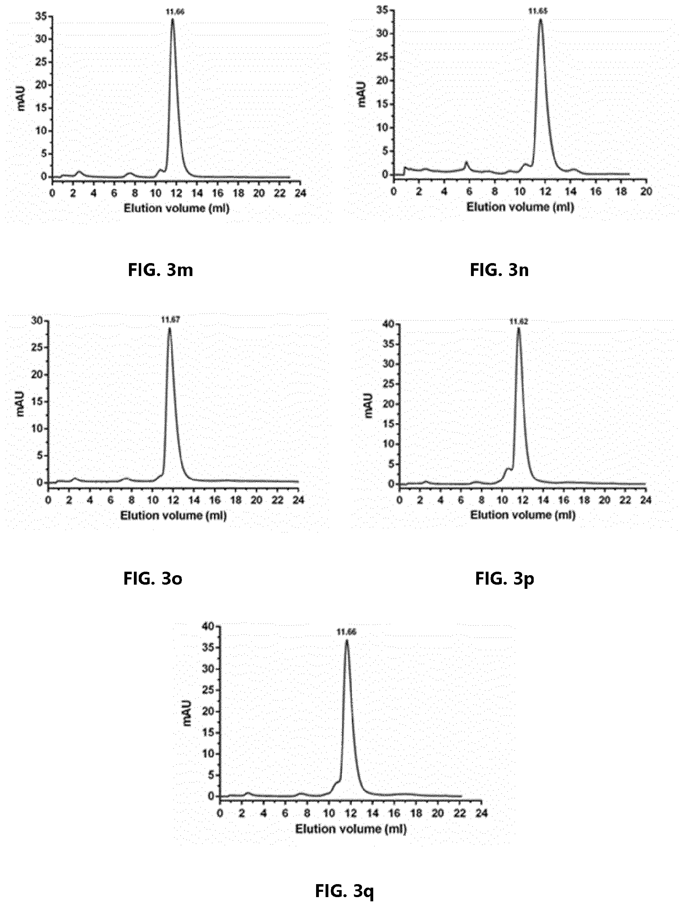

Figs. 3a-3q: the amino acid sequence set forth in SEQ ID NO: 1, wherein X0 is L; X1 is R; X2 is H; X3 is selected from the amino acid P, N, T, I, S, M, Q, R, L, G, V, A, E, D, Y, F, or H, respectively; X4 is T; and X5 is M. By SEC, IgG1 Fc mutants are detected to be present as monomers, respectively (Fig. 3a corresponds to the case where X3 is the amino acid P, Fig. 3b corresponds to the case where X3 is the amino acid N, Fig. 3c corresponds to the case where X3 is the amino acid T, Fig. 3d corresponds to the case where X3 is the amino acid I, Fig. 3e corresponds to the case where X3 is the amino acid S, Fig. 3f corresponds to the case where X3 is the amino acid M, Fig. 3g corresponds to the case where X3 is the amino acid Q, Fig. 3h corresponds to the case where X3 is the amino acid R, Fig. 3i corresponds to the case where X3 is the amino acid L, Fig. 3j corresponds to the case where X3 is the amino acid G, Fig. 3k corresponds to the case where X3 is the amino acid V, Fig. 3l corresponds to the case where X3 is the amino acid A, Fig. 3m corresponds to the case where X3 is the amino acid E, Fig. 3n corresponds to the case where X3 is the amino acid D, Fig. 3o corresponds to the case where X3 is the amino acid Y, Fig. 3p corresponds to the case where X3 is the amino acid F, and Fig. 3q corresponds to the case where X3 is the amino acid H);

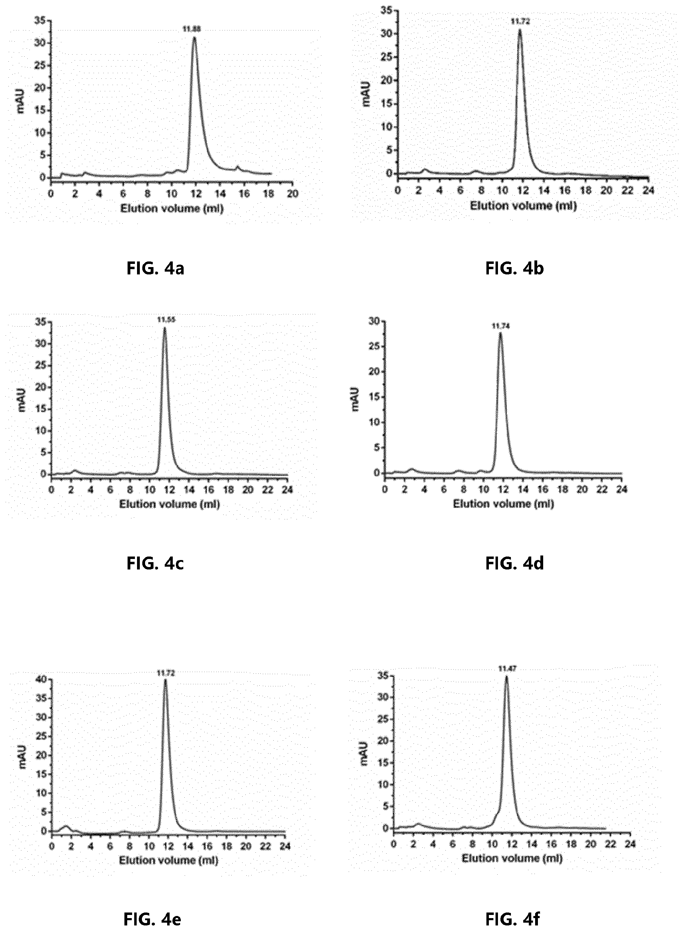

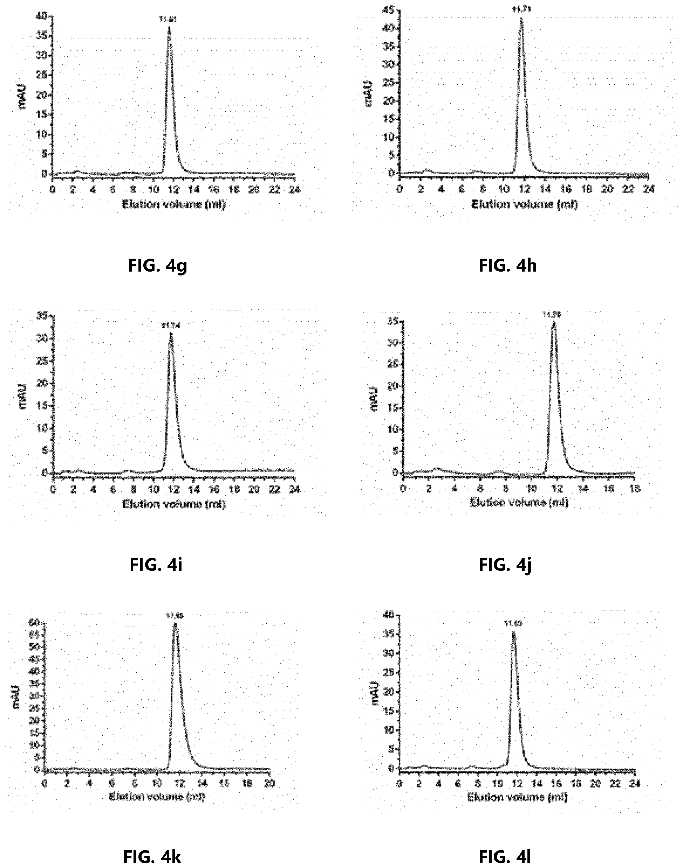

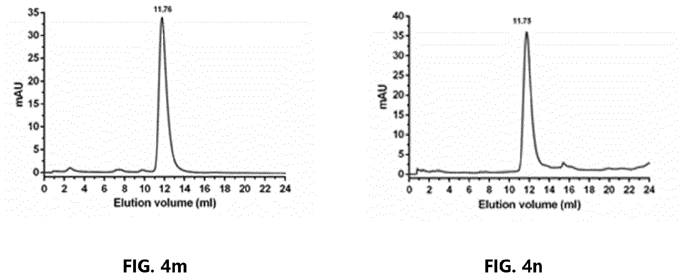

Figs. 4a-4n: the amino acid sequence set forth in SEQ ID NO: 1, wherein X0 is L; X1 is R; X2 is H; X3 is K; X4 is selected from the amino acid K, N, S, I, M, E, Q, L, V, A, H, D, Y, or F, respectively; and X5 is M. By SEC, IgG1 Fc mutants are detected to be present as monomers, respectively (Fig. 4a corresponds to the case where X4 is the amino acid K, Fig. 4b corresponds to the case where X4 is the amino acid N, Fig. 4c corresponds to the case where X4 is the amino acid S, Fig. 4d corresponds to the case where X4 is the amino acid I, Fig. 4e corresponds to the case where X4 is the amino acid M, Fig. 4f corresponds to the case where X4 is the amino acid E, Fig. 4g corresponds to the case where X4 is the amino acid Q, Fig. 4h corresponds to the case where X4 is the amino acid L, Fig. 4i corresponds to the case where X4 is the amino acid V, Fig. 4j corresponds to the case where X4 is the amino acid A, Fig. 4k corresponds to the case where X4 is the amino acid H, Fig. 4l corresponds to the case where X4 is the amino acid D, Fig. 4m corresponds to the case where X4 is the amino acid Y, and Fig. 4n corresponds to the case where X4 is the amino acid F);
Fig. 5a: determination of the monomeric profile of an IgG2 Fc mutant by SEC detection;
Fig. 5b: determination of the monomeric profile of an IgG3 Fc mutant by SEC detection;
Fig. 5c: determination of the monomeric profile of an IgG4 Fc mutant by SEC detection;
Fig. 6a: an IgG1 Fc monomer is selected, i.e., an amino acid sequence set forth in SEQ ID NO: 2, and the monomeric profile is determined by SEC;
Fig. 6b: surface plasmon resonance (SPR) is used to detect the binding capacity of the novel IgG1 Fc monomer to the Fc ligand FcRn (each of the lines in Fig. 6b represents a novel IgG1 Fc monomer, which is 2-fold diluted from 200 nM to 6.25 nM, sequentially from top to bottom);
Fig. 7a verifies by SEC that a fusion of a nanobody and an IgG1 Fc monomer as constructed by an IgG hinge region linker is present as a monomer fusion protein;
Fig. 7b verifies by SEC that a fusion of a nanobody and an IgG1 Fc monomer as constructed by a GGGGS linker is present as a monomer fusion protein;
Fig. 7c verifies by SEC that a fusion of a nanobody and an IgG1 Fc monomer as constructed by a (GGGGS)2 linker is present as a monomer fusion protein;
Fig. 7d verifies by SEC that a fusion of a nanobody and an IgG1 Fc monomer as constructed by a (GGGGS)3 linker is present as a monomer fusion protein; and
Fig. 7e: bio-layer interferometry (BLI) is used to detect the binding capacity of the novel IgG1 Fc monomer fusion protein to antigen gp120 (each of the lines in Fig. 7 represents a novel IgG1 Fc monomer fusion protein, which is 2-fold diluted from 1 uM to 62.5 nM, sequentially from top to bottom).

### Detailed Description of Embodiments

In order to enable a person skilled in the art to better understand the solution of the present application, the technical solutions in embodiments of the present application will be described below clearly and completely in conjunction with examples. Obviously, the described embodiments are only some, rather than all, of the embodiments of the present application. Based on the embodiments of the present application, all other embodiments obtained by those of ordinary skill in the art without involving any inventive effort should belong to the scope of protection of the present application.

As used herein, the term "monomer" refers to a molecule that can undergo polymerization to provide structural units for the basic structure of a polymer. The process in which a large number of monomers are combined to form a polymer is called polymerization.

As used herein, the term "polypeptide" refers to a polymer in which monomers are amino acid residues linked together via amide bonds. When the amino acid is an α-amino acid, an L-optical isomer or a D-optical isomer can be used. As used herein, the term "polypeptide" or "protein" is intended to cover any amino acid sequence and includes modified sequences, such as glycoproteins. The term "polypeptide" is particularly intended to cover naturally occurring proteins, as well as recombinant or synthetic proteins.

As used herein, the term "amino acid" means one of the 20 naturally occurring amino acids or any unnatural analogues, which can be located in a specified position. The term "protein" herein means at least two covalently linked amino acids, including proteins, polypeptides, oligopeptides, and peptides. A protein can be composed of naturally occurring amino acids and peptide bonds, or of synthetic peptide mimetic structures, which are called "analogues". Therefore, the term "amino acid" or "peptide residue" used herein means naturally occurring and synthetic amino acids. By way of example, for the purpose of the present invention, homophenylalanine, citrulline and norleucine are considered as amino acids for the purpose of the present invention. The term "amino acid" also includes imino acid residues such as proline and hydroxyproline. A side chain may be in the (R) or (S) configuration. In a preferred embodiment, an amino acid exists in the (S) or L-configuration. If non-naturally occurring side chains are used, non-amino acid substitutions can be used, for example, to prevent or delay *in vivo* degradation.

As used herein, the term "CH2 or CH3 domain" means a polypeptide derived from the CH2 or CH3 domain of an immunoglobulin, wherein the CH2 or CH3 domain molecule comprises at least one CDR or a functional fragment thereof; or may further comprise an additional amino acid sequence, such as a complete hypervariable loop; and may have at least a portion of one or more β strands replaced by CDR or a functional fragment thereof; or the CH2 or CH3 domain contains one or more mutations in the loop region of the molecule. The "loop region" in the CH2 or CH3 domain refers to a protein part located between β strand regions (for example, each CH2 domain contains 7 β sheets, A to G, oriented from N- to C-terminus). The sizes of the CH2 and CH3 domain molecules are small, usually less than 15 kD. The size thereof may vary depending on the length of the CDR/hypervariable amino acid sequence inserted into the loop region, or the number of CDRs inserted, and whether another molecule (such as an effector molecule or a label) binds to the CH2 or CH3 domain. The CH2 or CH3 domain molecule may be glycosylated or non-glycosylated. For example, a recombinant CH2 or CH3 domain may be expressed in a suitable mammalian cell to allow glycosylation of the molecule.

As used herein, the term "Fc fusion" means a protein in which one or more polypeptides are operably linked to an Fc region or a derivative thereof. The Fc fusion herein has the same meaning as the terms "immunoadhesin", "Ig fusion", "Ig chimera" and "receptor globulin" (sometimes with dashes) used in the prior art (Chamow et al., 1996, Trends Biotechnol 14: 52-60; and Ashkenazi et al., 1997, Curr Opin Immunol 9: 195-200).

The term "fusion partner" herein is a non-Fc part of an Fc fusion.

As used herein, the term "N-terminus", also known as amino terminus, NH2-terminus, N-terminus or amine terminus, is the start of a protein or polypeptide and refers to a free amine group (-NH2) located at the terminus of the polypeptide. The term "C-terminus", also known as carboxyl terminus, carboxyl terminus, C-terminal tail, C-terminus or COOH-terminus, is the end of a protein or polypeptide and is terminated by a free carboxyl (-COOH) terminus.

As used herein, the term "toxin" means a molecule that is cytotoxic to cells. Toxins include but are not limited to abrin, ricin, *Pseudomonas* exotoxin (PE), *diphtheria* toxin (DT), botulinum toxin, saporin, limitin or gelonin, or their modified toxins. For example, PE and DT are highly toxic compounds that usually lead to death by hepatotoxicity. However, by removing the natural targeting component (e.g., domain Ia of PE or B chain of DT) of the toxin and replacing it with a different targeting component, PE and DT can be modified to be used as immunotoxins, such as monomeric Fc, CH2 or CH3 domain molecules.

The term "immune cell" used herein refers to all cells involved in immune responses, and especially to lymphocytes that can recognize antigens and produce specific immune responses, etc. It mainly includes T lymphocytes, B lymphocytes, monocytes, macrophages, granulocytes, mast cells, accessory cell, their precursor cells, etc., and is a functional unit of the immune system. Depending on the function, immune cells can be divided into nonspecific immune cells, specific immune cells, and antigen presenting cells. Nonspecific immune cells include macrophages, neutrophils, natural killer cells, mast cells, etc. Specific immune cells include T cells and B cells, and antigen presenting cells include dendritic cells, macrophages, B cells, etc.

As used herein, the term "detectable label" means a detectable compound or composition that is directly or indirectly conjugated with another molecule, such as an antibody, an Fc domain or a CH2 or CH3 domain molecule, to facilitate the detection of the molecule. Specific non-limiting examples of labels include fluorescent labels, enzymatic ligation and radioisotopes.

The term "antibody" herein means a protein composed of one or more polypeptides encoded by all or some of generally well-established immunoglobulin genes. The well-established immunoglobulin genes, for example, in humans, includes kappa (κ), lambda (λ) and heavy chain loci, which contain numerous variable region genes, as well as constant region genes mu (µ), delta (δ), gamma (γ), epsilon (ε) and alpha (α) which respectively encode IgM, IgD, IgG, IgE and IgA isoforms. The antibody herein is meant to include full-length antibodies and antibody fragments, as well as natural antibodies from any organism, engineered antibodies, or antibodies produced by recombination for experimental, therapeutic or other purposes as further specified below. The term "antibody" includes antibody fragments well known in the art, such as Fab, Fab', F(ab')2, Fv, scFv or other antigen binding subsequences of antibodies, or antibody fragments produced by modifying intact antibodies or those synthesized by recombinant DNA technologies. The term "antibody" includes monoclonal and polyclonal antibodies. The antibody may be an antagonist, an agonist, a neutralizing antibody, an inhibitory antibody or an irritating antibody. The antibody of the present invention can be a non-human antibody, a chimeric antibody, a humanized antibody, or a fully human antibody.

Specifically included in the definition of "antibody" are non-glycosylated antibodies. Preferably, the term "non-glycosylated antibody" used herein means an antibody lacking sugar attachment at position 297 of the Fc region, wherein the numbering method is in accordance with the EU system of Kabat. A non-glycosylated antibody may be a deglycosylated antibody, which is an antibody from which an Fc sugar has been removed, for example, either chemically or enzymatically. Alternatively, the non-glycosylated antibody may be a non-glycosylated or un-glycosylated antibody, which is an antibody without Fc sugar expression, for example, by mutating one or more residues encoding glycosylation type or by expression in organisms such as bacteria that do not attach a sugar to a protein.

The term "IgG" used herein means a polypeptide belonging to the antibody class, which is encoded by a generally well-established immunoglobulin γ gene. In humans, this class includes IgG1, IgG2, IgG3, and IgG4. In mice, this class includes IgG1, IgG2a, IgG2b, and IgG3. The term "immunoglobulin (Ig)" herein means a protein consisting of one or more polypeptides substantially encoded by immunoglobulin genes. The immunoglobulin includes but is not limited to antibodies. The immunoglobulin may have many structural types, including but not limited to full-length antibodies, antibody fragments, and single immunoglobulin domains. The known Ig domains in the antibody class of IgGs are VH, Cγ1, Cy2, Cy3, VL, and CL.

As used herein, the term "antigen" means a compound, composition or substance that can stimulate antibody production or T cell responses in animals, including a composition to be injected or absorbed into animals, which may be proteins, sugars, lipids, or other pathogens.

As used herein, the term "cytokine" is meant to be used as a generic name for various soluble proteins and peptides, which act as humoral regulators at nano- to picomolar concentrations and regulate the functional activities of individual cells and tissues under normal or pathological conditions. These proteins also directly mediate interactions between cells and regulate processes occurring in the extracellular environment.

As used herein, the term "nucleic acid" means a polymer composed of nucleotide units (ribonucleotides, deoxyribonucleotides, related naturally occurring structural variants and their synthetic non-naturally occurring analogues) via phosphodiester bonds. Therefore, the term includes nucleotide polymers, in which nucleotides and bonds therebetween include non-naturally occurring synthetic analogues, such as but not limited to, thiophosphates, aminophosphates, methylphosphates, chiral methylphosphates, 2'-O-methylribonucleotides, peptide nucleic acids (PNAs), etc. For example, these polynucleotides can be synthesized using an automatic DNA synthesizer. The term "oligonucleotide" generally refers to a short polynucleotide, usually no more than about 50 nucleotides. It should be understood that when the nucleotide sequence is represented by a DNA sequence (i.e., A, T, G, and C), this also includes an RNA sequence in which "T" is replaced by "U" (i.e., A, U, G, and C).

Herein, conventional symbols are used to describe nucleotide sequences: the left-hand end of a single-stranded nucleotide sequence is the 5' end; and the left-hand direction of a double-stranded nucleotide sequence is called 5' direction. The direction in which 5' to 3' nucleotides are added to the newborn RNA transcript is called the transcription direction. A DNA strand with the same sequence as mRNA is called a coding strand.

As used herein, the term "plasmid" means a plasmid artificially constructed on the basis of a natural plasmid to adapt to laboratory operations. A nucleic acid molecule can be introduced into a host cell, thereby producing a transformed host cell. A vector may include a nucleic acid sequence that allows it to replicate in a host cell, such as origin of replication, and may also include one or more selectable marker genes and other genetic elements known in the art.

As used herein, the term "host cell", also called recipient cell, refers to a host cell that receives a foreign gene during transformation and transduction (infection).

As used herein, the term "pharmaceutically acceptable carrier" means a conventional pharmaceutically acceptable carrier. Remington's Pharmaceutical Sciences, EW Martin, Mack Publishing Co., Easton, Pa., 15th edition (1975), describes compositions and preparations suitable for drug delivery of one or more therapeutic compounds or molecules (such as one or more antibodies), and additional agents.

As used herein, the "diagnosis" of a disease refers to the diagnosis of a condition and the development thereof in a patient after examination. The "prevention" of a disease refers to inhibiting the complete development of the disease. The term "treatment" refers to therapeutic intervention to improve the signs or symptoms of a disease or a pathological condition after it begins to develop.

The term "administration" herein means selecting an appropriate route to introduce the substance into a subject. For example, if the selected route is intravenous, the composition is administered by introducing the substance into the vein of the subject.

The term "prophylactically/therapeutically effective dose" herein means an amount of a specific agent sufficient to achieve a desired effect in a subject treated with the agent. An exact dosage will depend on the purpose of treatment and can be determined by those skilled in the art by using well-known techniques. The dosage range can be 0.01-100 mg/kg body weight or more, such as 0.1, 1, 10, or 50 mg/kg body weight, preferably 1-10 mg/kg. As is well known in the art, in terms of the degradation of an antibody or Fc fusion, systemic or local drug delivery and new protease synthesis rate, as well as age, weight, general health, sex, diet, administration time, drug interaction, and the severity of the disease, adjustment may be necessary and can be determined by those skilled in the art by means of conventional experimental methods. Such agents include the monomeric Fc domain molecules described herein. In one non-limiting example, this may be the amount of an HIV-specific monomeric Fc domain (or HIV-specific CH3 domain molecule) for preventing, treating or ameliorating HIV infection. Ideally, a therapeutically effective amount of antibody is an amount sufficient to prevent, treat or ameliorate an infection or disease, such as that ascribable to HIV infection in a subject, without causing significant cytotoxic effects in the subject. A therapeutically effective amount of an agent for preventing, ameliorating and/or treating a subject will depend on the subject being treated, the type and severity of the indisposition, and the mode of administration of a therapeutic composition.

As used herein, the term "β strand" means a segment of polypeptide chain, usually 3 to 10 amino acids long, with the skeleton in an almost completely extended conformation. Generally, the β strand is a single continuous amino acid segment that takes this extended conformation, and involves hydrogen bonding with the backbone of at least one other chain, so that they form a β sheet. In β sheets, most β strands are arranged adjacent to other chains and form a wide hydrogen bond network with their neighbours, wherein the N-H groups in one chain skeleton form hydrogen bonds with the C=O groups in the adjacent strand of chain skeleton. In the fully extended β strand, the continuous side chain points straight up, then straight down, then straight up, etc. Adjacent β strands in the β sheet are aligned in a line such that Cα atoms thereof are adjacent, and the side chains thereof point in the same direction. The "pleated" appearance of the β strands are derived from tetrahedral chemical bonding of Cα atoms; for example, if the side chains point directly upward, the bond with Cv is necessarily slightly downward, because the bond angle thereof is about 109.5°. The distance between Cai and Cαi+2 caused by pleating is about 6 Å, instead of 7.6 Å (2x3.8 Å) as expected by two fully extended trans peptide virtual bonds. The "lateral" distance between adjacent Cα atoms in the hydrogen-bonded β strands is about 5 Å.

As used herein, the term "complementarity determining region (CDR)" means the region in which amino acid residues in the antibody variable region are more prone to variation in terms of the composition and arrangement order, also known as hypervariable region. There are three hypervariable regions (HVRs) in the V regions of the L chain and H chain, which can form precise complementarity with antigenic determinants in spatial structure. Each antigen receptor comprises six hypervariable loops: H1, H2, H3, L1, L2, and L3. For example, the H1 loop contains CDR1 of the heavy chain, and the L3 loop contains CDR3 of the light chain. CH2 and CH3 domain molecules may include transplanted amino acids from antibody variable domains. The transplanted amino acids include at least a portion of the CDR. The transplanted amino acids may further include an additional amino acid sequence, such as a complete hypervariable loop. As used herein, a "functional fragment" of a CDR is at least a portion of the CDR that retains the capacity to bind a specific antigen.

As used herein, the term "Fc receptor" means a molecule, preferably a polypeptide, from any organism that binds to the Fc region of an antibody to form an Fc receptor complex. Fc receptors include but are not limited to FcyR, FcyR, FcyR, FcRn, C1q, C3, mannan-binding lectin, mannose receptor, staphylococcal protein A, staphylococcal protein G, and virus FcyR. Fc receptors also include Fc receptor homologues (FcRHs). It is a family of Fc receptors homologous to FcyR (Davis et al., 2002, Immunological Reviews 190: 123-136). Fc receptors can also include undiscovered molecules that can bind Fc.

As used herein, the terms "Fc", "Fc region", "Fc monomer polypeptide" and the like include polypeptides comprising an antibody constant region other than the first constant region of the immunoglobulin domains. Therefore, Fc refers to the last two constant regions of IgA, IgD, and IgG immunoglobulin domains, the last three constant regions of IgE and IgM immunoglobulin domains, and a flexible hinge linking the N-terminus of these domains. For IgA and IgM, Fc can comprise J chain. For IgG, Fc comprises immunoglobulin domains Cy2 and Cy3 and a hinge between Cγ1 and Cy2. Although the boundary of Fc region can be changed, the human IgG heavy chain Fc region is usually defined as containing residue C226 or P230 at its carboxyl terminus, in which the numbering method is in accordance with an EU indexing method of Kabat. Fc can refer to an isolated region, or the region in the context of antibodies, antibody fragments, or Fc fusions. Fc can be an antibody, an Fc fusion, or a protein or protein domain containing Fc. Particularly preferred are Fc variants, which are non-naturally occurring Fc variants.

The term "isolated" herein means that a biological component (such as a nucleic acid molecule or a protein) has been substantially separated or purified from other biological components naturally occurring with respect to the component (such as other biological components in cells), such as other chromosomal and extrachromosomal DNAs, RNAs, and proteins, including other antibodies. The nucleic acid and protein that have been "isolated" include nucleic acids and proteins purified by standard purification methods. An isolated antibody is an antibody that has been substantially separated or purified from other proteins or biological components, thereby maintaining its antigen specificity. The term also includes nucleic acids and proteins (including Fc domains, CH2 domains, and CH3 domain molecules) prepared by recombinant expression in host cells, as well as chemically synthesized nucleic acids or proteins or fragments thereof.

As used herein, the term "linker" means a molecule or a group of molecules (such as a monomer or a polymer) that link two molecules, which usually puts the two molecules in a preferred configuration. A variety of strategies can be used to covalently link Fc to a fusion partner to produce an Fc fusion.

As used herein, the term "residue" means the position in a protein along with the amino acid identity associated therewith. For example, aspartic acid 297 (also known as Asn297 and N297) is a residue in human antibody IgG1.

As used herein, the term "variable region" means an immunoglobulin region containing one or more Ig domains substantially encoded by any Vκ, Vλ and/or VH genes, which constitute immunoglobulin κ, λ and heavy chain loci, respectively.

As used herein, the term "nanobody" means an engineered CH2 or CH3 domain molecule that specifically binds to an antigen and partially or completely retains at least one Fc binding function, e.g., binding to FcRn. CH2 and CH3 domain molecules engineered to bind antigens are the smallest known human antibody domain molecules that specifically bind antigens.

As used herein, the term "encoding" means the inherent property of a specific nucleotide sequence in a polynucleotide, such as a gene, a cDNA or an mRNA, to serve as a template for the synthesis of other polymers and macromolecules by biological processes with either a defined nucleotide sequence or a defined amino acid sequence, and the biological properties resulting therefrom. Therefore, if the transcription and translation of mRNA produced from this gene result in a protein in cells or other biological systems, the gene encodes the protein. A coding strand, the nucleotide sequence of which is identical to mRNA and is usually provided in a sequence listing, and a non-coding strand, used as a transcription template, for a gene or a cDNA, can be referred to as encoding the protein or other product of that gene or cDNA. Unless otherwise specified, the term "nucleotide sequence encoding an amino acid sequence" includes all nucleotide sequences which are degenerate with each other and encode the same amino acid sequence. A nucleotide sequence that encodes a protein or an RNA may also include introns.

As used herein, the term "operably linked" means that when a first nucleic acid sequence is in a functional relationship with a second nucleic acid sequence, the first nucleic acid sequence is operably linked to the second nucleic acid sequence. For example, if a promoter affects the transcription or expression of a coding sequence, the promoter is operably linked to the coding sequence. Usually, the operably linked DNA sequence is continuous, and when necessary, two protein coding regions are linked in the same reading frame.

The term "autoimmune disease" herein refers to a disease in which the immune system generates an immune response (e.g., a B cell or T cell response) against some antigens (i.e., autoantigens) of a normal host and then causes damage to tissues. The autoantigen may be derived from a host cell, or may be derived from a symbiotic organism, such as a microorganism (called a symbiotic organism) that normally colonizes the mucosal surface. Autoimmune diseases affecting mammals include but are not limited to rheumatoid arthritis, juvenile pauciarthritis, collagen-induced arthritis, adjuvant-induced arthritis, Sjögren's syndrome, multiple sclerosis, experimental autoimmune encephalomyelitis, inflammatory bowel disease (e.g., Crohn's disease and ulcerative colitis), autoimmune gastric atrophy, pemphigus vulgaris, psoriasis, vitiligo, type 1 diabetes, non-obese diabetes, myasthenia gravis, Graves' disease, Hashimoto's thyroiditis, sclerosing cholangitis, sclerosing sialadenitis, systemic lupus erythematosus, autoimmune thrombocytopenic purpura, Goodpasture's syndrome, Addison's disease, systemic sclerosis, polymyositis, dermatomyositis, autoimmune hemolytic anemia, pernicious anemia, etc.

As used herein, the term "binding capacity" means the strength of binding between a binding site and a ligand (for example, between an antibody, an antigen-binding Fc domain or an antigen-binding CH3 domain and an antigen or an epitope). The affinity of binding site X to ligand Y is expressed by the dissociation constant (Kd), and the dissociation constant (Kd) is the Y concentration required to occupy half of the binding sites of X present in a solution. A lower (Kd) indicates a stronger or higher affinity interaction between X and Y, and requires a lower concentration of ligands to occupy sites. In general, the binding affinity may be influenced by changes, modifications and/or substitutions of one or more amino acids in an epitope recognized by a paratope (a molecular part that recognizes the epitope). Binding affinity can be the affinity of an antibody to an antigen.

Unless otherwise specified, all the technical and scientific terms used herein have the same meaning as commonly understood by those of ordinary skill in the field to which the present disclosure belongs. Unless otherwise explicitly specified in the context, the singular terms "a", "an", and "the" include plural indicators. It is to be understood that all base sizes or amino acid sizes given for nucleic acids or polypeptides, as well as all molecular weights or molecular weight values, are approximate and provided for description. Although methods and materials similar or equivalent to those described herein can be used in the practice or testing of the present disclosure, suitable methods and materials are described below. The term "comprise" means "include". All publications, patent applications, patents and other references mentioned herein are incorporated by reference in their entirety. In case of conflict, the present description (including the explanation of terms) shall prevail. In addition, the materials, methods and examples are only illustrative and not restrictive.

The standard recombinant DNA technologies and molecular cloning technologies used in the examples are well known in the art (Ausubel, F. M. et al., Current Protocols in Molecular Biology, published by Greene Publishing Assoc. and Wiley-Interscience), and the materials and methods suitable for microbial growth are well known in the art. Main chemical and biochemical reagents are purchased from KAPA Biosystems, New England Biolabs, TransGen Biotech, Thermo Fisher Scientific, OMEGA bio-tek, etc.

The present invention will be illustrated in detail below in conjunction with specific examples:

### Example 1. Screening and judgement of IgG Fc monomer polypeptide

Mutation was performed in the constant region of IgG1 (genetically synthesized by GENEWIZ, Inc.). According to a new synthetic biology technology developed by our laboratory, full coverage mutation was performed on amino acid sites critical for the formation of an Fc dimer (Leu-351, Thr-366, Leu-368, Pro-395, and Lys-409). A random mutation site (Met-428) related to FcRn binding was also included (Met). Based on this technology, we could simultaneously mutate the above sites to construct an IgG Fc mutant antibody library with a library size of 6.4 million. On the basis of the above library construction, a new method of antigenic solid-phase adsorption screening was innovatively developed, and soluble FcRn protein (purchased from Thermo Fisher) was used to screen IgG1 Fc monomer.

The specific operation was as follows:
A. 3% MPBS was prepared, a 96-well ELISA plate coated with antigen FcRn overnight was centrifuged at 10,000 rpm for 5 min, the supernatant was discarded, and the plate was blocked with 3% MPBS at 37°C for 1 h;
B. the plate was washed. Phage was re-suspended with 3% MPBS. The phage was added to the above 96-well ELISA plate, which was incubated at 37°C for 3 h;
C. the 96-well ELISA plate was washed with PBST (pH 6.0) five times, 100 uL of TG1 cells was added, which was incubated at 37°C for 30 min, and the cells were collected. 80 uL of an elution buffer (35 uL of triethylamine added to 2.5 mL of water) was added to each well and incubated at 37°C for 10 min. 0.4 mL of the eluent was mixed with 0.2 mL of Tris-HCl pH 7.5, and 3 mL of TG1 cells were added and incubated at 37°C for 45 min;
D. ampicillin and glucose were added to the infected TG1 cells, and the cells were cultured at a temperature of 37°C at 250 rpm for 1.5 h; and
E. 50 uL of helper phage was added, incubated at 37°C for 30 min, and centrifuged at 5,000 rpm for 10 min. A medium in which ampicillin and kanamycin were added was used for re-suspension to 10 mL of 2YT and incubated at 37°C at 250 rpm.

The above process was repeated for 4 rounds. Antibody enrichment was detected by polyclonal phage enzyme-linked immunosorbent assay (ELISA, using an instrument from BioTek Instruments, US). According to the results of polyclonal phage ELISA, the library was enriched significantly. Therefore, the phage obtained from the last round of screening was selected to infect TG1 cells (purchased from Thermo Fisher), clones were randomly selected for monoclonal phage ELISA, further sequencing was carried out to identify the enriched IgG1 Fc mutant, and size exclusion chromatography (SEC, using an instrument from GE Healthcare) was used to judge monomers. The analysis of sequencing and SEC results was as follows:
An amino acid sequence set forth in SEQ ID NO: 1, wherein when X0 was L; X1 was selected from the amino acid C, G, S, L, N, D, F, I, V, Y, Q, K, E, M, or T, respectively; X2 was H; X3 was K; X4 was T; and X5 was M, IgG1 Fc mutants were detected to be present as monomers by SEC, respectively (as in Figs. 1a-1o); wherein when X0 was L; X1 was selected from H or A, respectively; X2 was H; X3 was K; X4 was T; and X5 was M, the Fc mutants were present as a dimer; and wherein when X0 was L; X1 was P; X2 was H; X3 was K; X4 was T; and X5 was M, the mutant was expressed as an inclusion body.

An amino acid sequence set forth in SEQ ID NO: 1, wherein when X0 was L; X1 was R; X2 was the amino acid L, Q, N, D, Y, R, C, G, S, F, T, I, V, A, K, or M, respectively; X3 was K; X4 was T; and X5 was M, IgG1 Fc mutants were detected to be present as monomers by SEC, respectively (as in Figs. 2a-2p); wherein when X0 was L; X1 was R; X2 was E; X3 was K; X4 was T; and X5 was M, the mutant was detected to be present as a dimer by SEC; and wherein when X0 was L; X1 was R; X2 was P; X3 was K; X4 was T; and X5 was M, the mutant was expressed as an inclusion body.

An amino acid sequence set forth in SEQ ID NO: 1, wherein when X0 was L; X1 was R; X2 was H; X3 was selected from the amino acid P, N, T, I, S, M, Q, R, L, G, V, A, E, D, Y, F, or H, respectively; X4 was T; and X5 was M, IgG1 Fc mutants were detected to be present as monomers by SEC, respectively (as in Figs. 3a-3q); and wherein when X0 was L; X1 was R; X2 was H; X3 was C; X4 was T; and X5 was M, the mutant was detected to be present as a dimer by SEC.

An amino acid sequence set forth in SEQ ID NO: 1, wherein when X0 was L; X1 was R; X2 was H; X3 was K; X4 was selected from the amino acid K, N, S, I, M, E, Q, L, V, A, H, D, Y, or F, respectively; and X5 was M, the IgG1 Fc mutant was detected to be present as a monomer by SEC (as in Figs. 4a-4n); and wherein when X0 was L; X1 was R; X2 was H; X3 was K; X4 was selected from the amino acid R, P, G, or C, respectively; and X5 was M, the protein soluble expression of the Fc mutants was seriously affected.

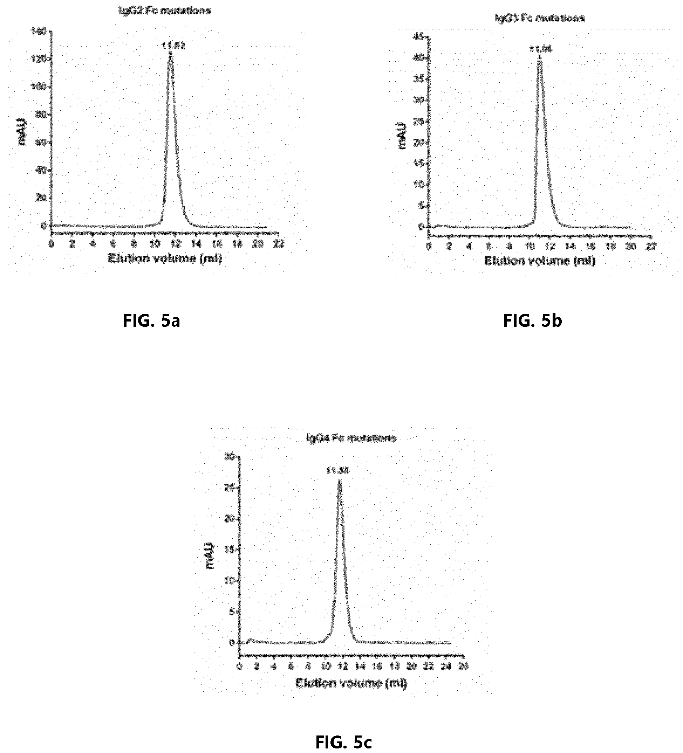

Based on the above-mentioned sites of IgG1 and the high homology of the constant regions of IgG2, IgG3 and IgG4 to that of IgG1, the key sites affecting the dimerization of the constant region Fc were analyzed, and it was found that the sites Thr-366, Leu-368, Pro-395, and Lys-409 were crucial for the formation of dimers, and the site Met-428 did not improve the affinity between monomer Fc and FcRn. Therefore, in order to reduce the immunogenicity of the mutant to the greatest extent and benefit the subsequent clinical application, we selected these four key mutation sites (Thr-366, Leu-368, Pro-395, and Lys-409) to synthesize constant region point mutation genes of IgG2, IgG3, and IgG4 (genetically synthesized by GENEWIZ, Inc.) (the amino acid sequence encoded by the IgG2 mutant gene was set forth in SEQ ID NO: 3, the amino acid sequence encoded by the IgG3 mutant gene was set forth in SEQ ID NO: 4, and the amino acid sequence encoded by IgG4 mutant gene was set forth in SEQ ID NO: 5), and the gene was enzymatically linked to prokaryotic expression vector pComb3x (the vector was donated by Dr. Dennis Burton, The Scripps Research Institute) to construct a prokaryotic expression vector. The preparation of prokaryotic soluble expression was basically carried out according to the literature (YING, Tianlei et al., Journal of Biological Chemistry, 2012, 287 (23): 19399-19408), and the results showed that the protein had an extremely high yield of soluble expression in *Escherichia coli.* The data of size exclusion chromatography showed that the protein was still a single-component pure monomer molecule (as shown in Figs. 5a-5c).

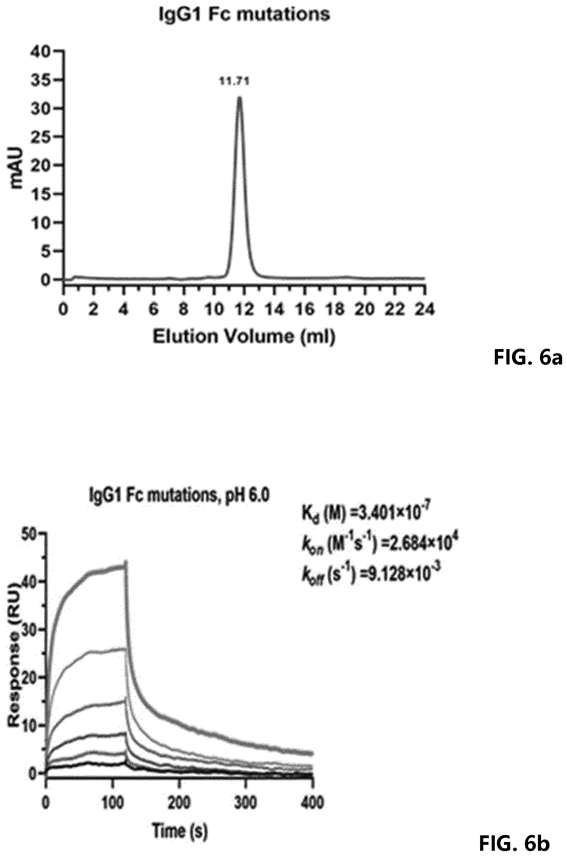

In order to verify the characteristics of pH-dependent binding of the IgG Fc monomer to FcRn, an IgG1 Fc mutant with high soluble expression in prokaryotic cells was selected (the amino acid sequence set forth in SEQ ID NO: 2) was selected. It was confirmed by size exclusion chromatography that it existed as a monomer (as in Fig. 6a), and it was confirmed by surface plasmon resonance (SPR) that it still retained the characteristics of pH-dependent binding of antibody IgG Fc to FcRn (as in Fig. 6b).

### Example 2. Experiment of fusion of IgG1 Fc monomer with nanobody

Separately with an IgG1 Fc monomer and a nanobody (nanobodies related to virus etc. as screened by the laboratory of the inventors, e.g., anti-HIV-1 gp120 nanobody m36.4, the amino acid sequence of which was SEQ ID NO: 6, see the reference J Virol. 2014 Jan; 88 (2): 1125-39) as the template, two pairs of primers were designed respectively, and the IgG1 Fc monomer gene and the nanobody gene were amplified by PCR.

Reaction conditions: pre-denaturation at 95°C for 35 min, denaturation at 95°C for 1 min, annealing at 58°C for 1 min, extension at 72°C for 1 min, for 30 cycles, and then extension at 72°C for 10 min (the program could be adjusted depending on the gene length). The product amplified by PCR was analyzed by 1.0% agarose gel electrophoresis to obtain IgG1 Fc monomer gene and nanobody gene fragments.

The IgG1 Fc monomer gene and nanobody gene fragments were combined by overlapping extension PCR (SOE-PCR). Finally, 1.0% agarose gel electrophoresis analysis was carried out to determine the size of the fusion gene fragment.

After the fusion gene fragment and Pcom3x clone plasmid (donated by Dr. Dennis Burton, The Scripps Research Institute) were individually digested with sfil restriction endonuclease (purchased from New England BioLabs), the two fragments were linked using T4 DNA ligase (purchased from New England BioLabs) at 16°C overnight, competent TOP10 *Escherichia coli* was then transformed with the recombinant plasmid, positive single colonies were selected, and the plasmid was extracted and sent to the company (GENEWIZ, Inc.) for sequencing.

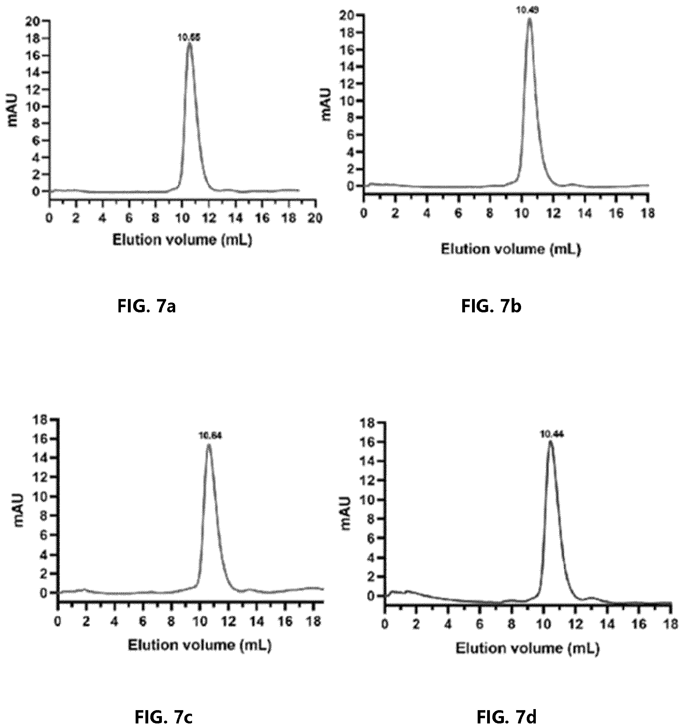

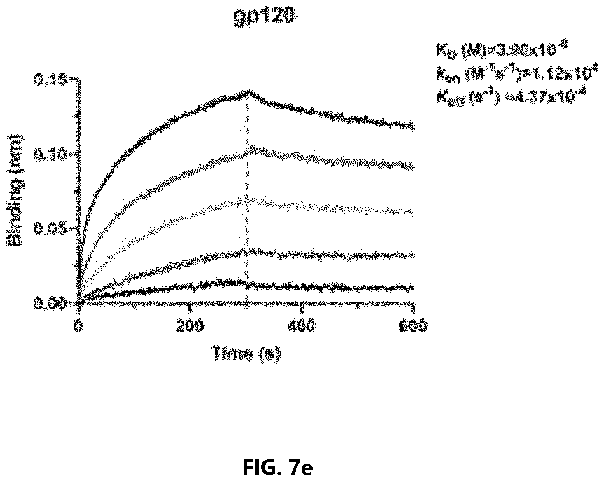

The above fusion gene was expressed in prokaryotic cells, specifically by transferring the plasmid into HB2151 competent cells (the preparation of the chemical competent cells, involving: (1) strain activation, wherein competent HB2151 cells were taken from a refrigerator at -70°C and separated by streaking on a 2YT plate;
(2) pre-culture of strain, wherein the corresponding single colony was selected from the 2YT plate, inoculated into 10 ml of a 2YT liquid medium, and cultured under shaking at 37°C overnight to the middle and late logarithmic growth phase;
(3) preparation of strain, wherein four bacterial suspensions were inoculated in four conical flasks containing 100 ml of 2YT liquid medium free of antibiotics at a ratio of 1 : 100, respectively, and cultured under shaking at 37°C for about 2.5 h until OD = 0.4-0.5; and
(4) preparation of competent cells, wherein: 1) the conical flask was placed in ice water for rapid cooling, and 100 mL of the culture solution was evenly divided into two parts and added to 50 mL centrifugal tubes, which were centrifuged at 4°C at 3000 rpm for 10 min; 2) the supernatant was discarded, and 30 mL of 0.1 M CaCl₂ solution was added, gently mixed until uniform, and left to stand in ice water for 30 min; 3) the supernatant was discarded, and 30 mL of 0.1 M CaCl₂ solution was added, pasteur pipet was used to mix the precipitate well by gentle pipetting, and the mixture was left to stand in ice water for 30 min; 4) the mixture was taken out of the ice water, centrifuged at 3,000 rpm at 4°C for 10 min, the supernatant was discarded and the excess liquid was gently sucked clean with a pipette, 0.5 mL of 0.1 M CaCl₂ solution containing 10% glycerol was added, the liquid was gently sucked up with the pasteur pipet, hit against the wall to make the precipitate evenly mixed, and placed on ice; 5) liquid nitrogen was poured into a small plastic box, and 0.5 ml centrifuge tubes were placed on a centrifuge tube rack, and then sub-packaged with trimmed pipette tips, wherein each centrifuge tube was sub-packaged with 40 µL of the suspension; and 6) the tubes were quickly capped, put into liquid nitrogen, quickly frozen, then put into bags marked with the type of the competent cells, the producer, and the time, and stored at -70°C.), and a single colony was selected from an overnight growing ampicillin dish, inoculated into SB bacterial culture solution, and expressed under IPTG induction conditions for 12-14 h at 30°C. Bacteria were harvested and purified by Protein G. It was found that the fusion protein could be efficiently expressed in a soluble manner in prokaryotic cells, and the data of size exclusion chromatography showed that the fusion protein was still a single-component pure monomer molecule (as shown in Figs. 7a, 7b, 7c, and 7d). The corresponding antigen was coated on an ELISA plate, and the fusion protein diluted at a concentration gradient was added for incubation. The binding capacity of the fusion protein of the novel IgG1 Fc monomer and nanobody to the corresponding antigen was detected by anti-FLAG tag antibody (purchased from Sigma). Bio-layer interferometry (BLI) combined with experiments showed that the fusion protein completely retained the antigen binding activity of the nanobody (Fig. 7e). The constructed fully humanized nanobody-IgG1 Fc monomer fusion protein was shown to have better feasibility.

### Example 3. Experiment of fusion of novel IgG Fc monomer with single-chain antibody

By means of genetic engineering, the expression gene of an IgG Fc monomer was inserted into an expression vector of a single-chain antibody (m912-scFv, Mol Cancer Ther. 2009 May; 8 (5): 1113-8), the IgG Fc monomer was linked to the C-terminus of the single-chain antibody, and four linkers, i.e., an IgG hinge region, GGGGS, (GGGGS)2, and (GGGGS)3, were separately inserted to construct a fusion protein expression vector. The expression and preparation of the fusion protein were basically carried out according to the literature (YING, Tianlei et al., Error! Hyperlink reference not valid. 2014; 6 (5): 1201-10).

By means of methods such as temperature-controlled circular dichroism spectroscopy (with an instrument purchased from Jasco International) and dynamic light scattering (DLS), the yield, stability and aggregation of the fusion proteins based on different linkers were further compared to determine the most suitable linker. As detected by methods such as ELISA and surface plasmon resonance (SPR), the fusion protein was measured for the binding capacity to the corresponding antigen and thermodynamic kinetic constants such as binding rate constant, dissociation rate constant, and affinity constant.

The results showed that the fusion protein of the IgG Fc monomer and the single-chain antibody had very strong binding capacity to the corresponding target protein. In addition, the target protein was fused with a specific transmembrane region by a genetic engineering method and transfected into HEK-293 cells (purchased from Thermo Fisher) to construct cells that over-expressed the target protein on the surface. As determined by flow cytometry, the fusion protein of the IgG Fc monomer and the single-chain antibody had strong binding capacity to the cells that expressed the target protein.

### Example 4. Experiment of fusion of IgG Fc monomer with immunotoxin

By means of genetic engineering, an IgG Fc monomer gene was recombined with *Pseudomonas aeruginosa* exotoxin PE38 gene (Gene ID: 3890513) (synthesized by Genscript) via (G4S)3 linker to construct a prokaryotic expression vector. The preparation of prokaryotic soluble expression was basically carried out according to the literature (YING, Tianlei et al., Journal of Biological Chemistry, 2012, 287 (23): 19399-19408). From the previous data of the inventors, it was found that the IgG Fc monomer could bind FcyRI with high affinity (YING, Tianlei et al., MAbs. 2014; 6 (5): 1201-10). U937 cells that over-expressed FcyRI on the surface and ordinary HEK-293F cells that did not express FcyRI on the surface as a negative control were constructed (see the reference: YING, Tianlei et al., MAbs. 2014; 6 (5): 1201-10), the two were incubated with 200 ul of PBSA and FITC-labeled mouse anti-human FcyRI secondary antibody (purchased from Invitrogen) on ice for 30 min, and by means of detection with flow cytometry, it was found that after the novel IgG1 Fc monomer was fused with immunotoxin protein PE38, the fused immunotoxin could specifically kill U937 cells that over-expressed FcyRI, but had no cytotoxicity to ordinary cells (HEK-293F cells that did not express FcyRI on the surface). The above results indicated that the fused immunotoxin could specifically kill the cells that overexpressed FcyRI on the surface.

### Example 5. Experiment of fusion of IgG Fc monomer with cytokine

By means of genetic engineering, an IgG Fc monomer gene was recombined with insulin gene (Gene ID: 3630) via a linker to construct a prokaryotic expression vector. The preparation of prokaryotic soluble expression was basically carried out according to the literature (YING, Tianlei et al., Journal of Biological Chemistry, 2012, 287 (23): 19399-19408), and the results showed that this fusion protein had an extremely high soluble expression yield in *Escherichia coli* and an extremely high thermodynamic stability. The data of size exclusion chromatography showed that the fusion protein was still a single-component pure monomer molecule. By pharmacokinetic detection in mice, it was found that the fusion protein of IgG Fc monomer and insulin had a half-life much longer than that of insulin itself.

### Example 6. Experiment relating to fusion of IgG Fc monomer with CD16A and gp140 protein binding region to construct bispecific antibody

By means of genetic engineering, anti-CD16A nanobody (n118, with the amino acid sequence SEQ ID NO: 7, from Patent CN 108101988 A) and HIV-1 virus gp140 protein binding region (md1.22, with the amino acid sequence SEQ ID NO: 8, from Dimiter S. Dimitrov's research group of US NIH) were respectively linked to the N-terminus and C-terminus of the novel IgG1 Fc monomer via the linkers DKTHTGP and (G4S)3.

The preparation of the soluble expression product of the bispecific long-acting antibody was basically carried out according to the literature (Ying, T. et al., Exceptionally potent neutralization of Middle East respiratory syndrome coronavirus by human monoclonal antibodies. J Virol 88, 7796-805 (2014).). Specifically, the plasmid was transferred into HB2151 competent cells, and a single colony was selected from an overnight growing ampicillin dish, inoculated into SB bacterial culture solution, and expressed under IPTG induction conditions for 12-14 h at 30°C. Bacteria were harvested and purified by Protein G. Circular dichroism spectrum (CD, with the instrument purchased from Jasco International) and dynamic light scattering (DLS, with the instrument purchased from Malvern Instruments Limited) showed that it had good thermal stability and polymerization stability. The results of ELISA and bio-layer interferometry (BLI, with the instrument purchased from Pall ForteBio) showed that the bispecific antibody had very strong binding to CD16A and gp140 protein. The results of detection by using an ADCC reporting system based on Jurkat T cells from Promega showed that the bispecific antibody had a very strong ADCC effect. 293T cells were co-transfected with an HIV-1 skeleton plasmid (Nature. 2014 Nov 6; 515 (7525): 138-42) (the reading frame of env gene in this plasmid was shifted but not expressed, and the nef gene was replaced by luciferase gene) and gp160 eukaryotic expression plasmid (Nature. 2014 Nov 6; 515 (7525): 138-42), and HIV-1 pseudoviruses were packaged. HEK-293 cells were co-infected with the bispecific antibody and the pseudoviruses. After a period of time of incubation, the content of luciferase (Promega) in the cell lysate was detected, and the results showed that the bispecific antibody had a strong pseudovirus neutralizing effect.

An IgG Fc monomer was fused with CD16A and gp140 protein binding region to construct a protein, i.e., a bispecific antibody, only fused with CD16A and gp140 protein binding region (n118-mD1.22). Pharmacokinetics were tested in C57BL/6 mice, with 7 mice in each group. 0.5 mg of the antibody was injected into the tail vein of each mouse, and blood samples were collected from the orbit of the mice at different time points for detection. The results showed that the bispecific antibody constructed by the fusion of the novel IgG1 Fc monomer with CD16A and gp140 protein binding region had a longer half-life, which could be as long as more than twenty hours.

In order to detect the developability of the bispecific long-acting antibody, the aggregation properties of the antibody was assessed by means of DLS (dynamic light scattering), and the thermal stability of the antibody was assessed by means of CD (circular dichroism spectrum). The results showed that the bispecific antibody was presented as a small particle antibody with high solubility and high thermal stability and had certain developability.

### Example 7. Combined use of IgG Fc monomer fusion protein and anti-HIV-1 antibody to inhibit HIV-1 virus

The bispecific antibody constructed by the fusion of the IgG Fc monomer with CD16A and gp140 protein binding region could be used in combination with an existing anti-HIV-1 antibody. In monkeys, a therapy with combined injection of the two antibodies could maintain the inhibition of human immunodeficiency virus type 1 (HIV-1) and reduce the viral load.

### Example 8. Fusion of IgG Fc monomer and hemophilia factor to prevent acute hemorrhage of hemophilia

By means of genetic engineering, an IgG Fc monomer was recombined with hemophilia B factor IX and hemophilia A factor VIII to construct an eukaryotic expression vector. The expression and preparation of the fusion protein were basically carried out according to the literature (YING, Tianlei et al., Journal of Biological Chemistry, 2012, 287 (23): 19399-19408). The expression results showed that the fusion protein had an extremely high soluble expression yield in eukaryotic cells and an extremely high thermodynamic stability. The results of the pharmacokinetic detection in mice showed that the plasma half-life of the novel IgG1 Fc monomer fusion recombinant protein was longer than that of the corresponding original molecule. It could have a good preventive effect on acute hemorrhage in a patient with hemophilia.

### Example 9. IgG Fc monomer fusion protein to neutralize HIV-1 virus

By means of genetic engineering, an IgG Fc monomer gene was recombined separately with an HIV-1 gp120-related nanobody and a gp140 protein binding region via a linker to construct a prokaryotic expression vector. The expression and preparation of the fusion protein were basically carried out according to the literature (YING, Tianlei et al., Journal of Biological Chemistry, 2012, 287 (23): 19399-19408). Prokaryotic soluble expression showed that this fusion protein had an extremely high soluble expression yield in *Escherichia coli* and an extremely high thermodynamic stability. As detected by methods such as ELISA and surface plasmon resonance (SPR), the fusion protein had a very strong binding capacity to the corresponding antigen of HIV-1. Pseudovirus neutralization experiments and pharmacokinetic detection in animals showed that the novel IgG1 Fc monomer fusion protein had a good neutralization activity and a prolonged half-life *in vivo.* It was promising as a candidate drug for subsequent treatment of HIV-1.

### Example 10. IgG Fc monomer fusion protein to detect HIV-1 virus

By means of genetic engineering, an IgG Fc monomer gene was recombined separately with an HIV-1 gp120-related nanobody and a gp140 protein binding region via a linker to construct a prokaryotic expression vector. The expression and preparation of the fusion protein were basically carried out according to the literature (YING, Tianlei et al., Journal of Biological Chemistry, 2012, 287 (23): 19399-19408). The fusion protein could be used as a primary antibody to detect HIV-1 infection by ELISA method and to qualitatively detect whether there was HIV-1 infection or not. On the premise of good quality control evaluation, it could be manufactured into a detection kit for a commercial product.

Although the embodiments of the present invention have been described in conjunction with the drawings, various modifications and variations can be made by those skilled in the art without departing from the spirit and scope of the present invention, and such modifications and variations all fall within the scope defined by the appended claims.

## Claims

1. An Fc monomer polypeptide comprising CH2 and CH3 domains, wherein the Fc monomer polypeptide comprises an amino acid sequence set forth in SEQ ID NO: 1, wherein X0 is L or S; X1 is any of amino acid C, G, S, L, N, D, F, I, V, Y, Q, K, E, M, T, or R; X2 is any of amino acid H, L, Q, N, D, Y, R, C, G, S, F, T, I, V, A, K, or M; X3 is any of amino acid P, N, T, I, S, M, Q, R, L, G, V, A, E, D, Y, F, H, or K; X4 is any of amino acid K, N, S, I, M, E, Q, L, V, A, H, D, Y, F, or T; and X5 is M or Y

2. The Fc monomer polypeptide according to claim 1, wherein the sequence of the Fc monomer polypeptide further comprises an amino acid sequence set forth in SEQ ID NO: 2.

3. The Fc monomer polypeptide according to claim 2, wherein the sequence of the Fc monomer polypeptide further comprises an amino acid sequence set forth in SEQ ID NO: 3.

4. The Fc monomer polypeptide according to claim 3, wherein the sequence of the Fc monomer polypeptide further comprises an amino acid sequence set forth in SEQ ID NO: 4.

5. The Fc monomer polypeptide according to claim 4, wherein the sequence of the Fc monomer polypeptide further comprises an amino acid sequence set forth in SEQ ID NO: 5.

6. An isolated CH3 domain, comprising:
amino acids at positions 113-217 in the amino acid sequence of SEQ ID NO: 1 as defined in claim 1; further comprising amino acids at positions 113-217 in the amino acid sequence of SEQ ID NO: 2 as defined in claim 2; further comprising amino acids at positions 113-217 in the amino acid sequence of SEQ ID NO: 3 as defined in claim 3; further comprising amino acids at positions 113-216 in the amino acid sequence of SEQ ID NO: 4 as defined in claim 4; and further comprising amino acids at positions 113-217 in the amino acid sequence of SEQ ID NO: 5 as defined in claim 5.

7. A fusion, formed from a fusion partner connected to the N-terminus and/or C-terminus of the peptide chain of the Fc monomer polypeptide according to one of claims 1 to 5 or the CH3 domain according to claim 6.

8. The fusion according to claim 7, wherein the fusion partner is at least one of a heterologous protein, an adhesion molecule, a nucleic acid molecule, a small molecule compound, a toxin, an immune cell, and a detectable label, wherein the heterologous protein is at least one of an antibody, an antigen, a cytokine, a soluble receptor or a target binding region thereof fusion, a biological enzyme, a peptide, and a protein domain.

9. The fusion according to claim 8, wherein the antigen is derived from an adeno-associated virus; and the immune cell is a chimeric antigen receptor T cell.

10. The fusion according to claim 9, further comprising an amino acid sequence set forth in SEQ ID NO: 6, an amino acid sequence set forth in SEQ ID NO: 7, or an amino acid sequence set forth in SEQ ID NO: 8.

11. A nucleic acid molecule encoding the Fc monomer polypeptide according to any one of claims 1 to 5, the CH3 domain according to claim 6, and/or the fusion according to any one of claims 7 to 10.

12. A plasmid comprising the nucleic acid molecule according to claim 11.

13. A host cell comprising the plasmid according to claim 12.

14. A pharmaceutical composition, comprising the Fc monomer polypeptide according to any one of claims 1 to 5, the CH3 domain according to claim 6, or the fusion according to any one of claims 7 to 10, and a physiologically or pharmaceutically acceptable carrier; preferably, further comprising a prophylactically or therapeutically effective dose of the nucleic acid molecule according to claim 11 or the plasmid according to claim 12, and a physiologically or pharmaceutically acceptable carrier.

15. A detection kit, comprising the fusion according to any one of claims 7 to 10, the nucleic acid molecule according to claim 11, or the plasmid according to claim 12.

16. Use of the detection kit according to claim 15 for detecting a pathogen or a tumor cell, preferably for detecting human immunodeficiency virus HIV.
